# EUROPEAN PATENT SPECIFICATION

(11) **EP 3 441 433 B1**
(45) Date of publication and mention of the grant of the patent: **17.06.2020**
(21) Application number: 18174933.4
(22) Date of filing: 10.01.2013
(51) Int. Cl.: C09D 5/16, B08B 17/00, B05D 3/10, B05D 5/08, A61L 29/08, A61L 33/00, A61L 29/06, B05D 7/02

(54) **MODIFICATION OF MEDICAL DEVICE SURFACES FOR FLUID AND SOLID REPELLENCY**
MODIFIKATION VON MEDIZINPRODUKT OBERFLÄCHEN ZUR ABWEISUNG FLÜSSIGER UND FESTER SUBSTANZEN
MODIFICATION DE SURFACES DES PRODUITS MÉDICAUX AUX FINS DE RÉSISTANCE AUX LIQUIDES ET AUX SOLIDES

(30) Priority: 10.01.2012 US 201261585059 P; 13.07.2012 US 201261671645 P; 13.07.2012 US 201261671442 P; 22.08.2012 US 201261692079 P
(43) Date of publication of application: 13.02.2019
(62) Divisional of application: 13705284.1
(73) Proprietor: President and Fellows of Harvard College, Cambridge, MA 02138 (US)
(72) Inventor: INGBER, Donald, Boston, Massachusetts 02118 (US); LESLIE, Daniel, C., Roslindale Massachusetts 02131-2752 (US); WATTERS, Alexander, L., Melrose, Massachusetts 02176 (US); SUPER, Michael, Lexington, Massachusetts (US); ALZENBERG, Joanna, Boston, Massachusetts 02111 (US); ALZENBERG, Michael, Boston, Massachusetts 02111 (US); KIM, Philseok, Arlington, Massachusetts 02474 (US); WATERHOUSE, Anna, Cambridge, Massachusetts 02138 (US)
(74) Representative: HGF Limited

(56) References cited:
- EP-A2- 0 166 998
- WO-A1-93/17077
- TAK-SING WONG ET AL: "Bioinspired self-repairing slippery surfaces with pressure-stable omniphobicity", NATURE, vol. 477, no. 7365, 1 January 2011 (2011-01-01), pages 443-447, XP055025132, ISSN: 0028-0836, DOI: 10.1038/nature10447

## Description

### CROSS-REFERENCE TO RELATED APPLICATIONS

This application claims priority to U.S. Provisional Patent Application No. 61/585,059 filed January 10, 2012 and U.S. Provisional Patent Application No. 61/692,079 filed August 22, 2012.

### STATEMENT CONCERNING GOVERNMENT RIGHTS IN

### FEDERALLY-SPONSORED RESEARCH

This invention was made with government support under Grant No. 5U01NS073474-03 awarded by the National Institutes of Health and under N66001-11-1-4180 awarded by the U.S. Department of Defense. The government has certain rights in this invention.

### TECHNICAL FIELD

The present disclosure relates generally to medical device surfaces that are transformed to reduce friction between, and prevent adhesion, adsorption, and deposition from liquids, semi-solids, and solids.

### BACKGROUND

There has been limited success in developing materials that prevent molecular or particulate adhesion, adsorption, deposition and biofouling of a variety of commercially available surfaces for use in medical devices, such as catheters, syringes, dialysis instruments, and for the prevention of blood coagulation or clotting, oil pipelines, food and cooking surfaces, and preventing ice adhesion and formation.

Synthetic surfaces have been made that consist of nano/microstructured substrates infused with fluid that is locked in place by a roughened or porous substrate to form a slippery interface capable of repelling liquids. However, such surfaces are limited to particular combinations of lubricating liquids and substrates having a certain roughness or porosity capable of retaining the lubricating fluid.

Silanization of glass and metal oxides is a general method for modifying a material to make its surface less or more attractive to the other substances. A thin perfluorocarbon ("PFC") layer (omniphobic or amphiphobic as discussed in the literature) is generated by silanization of surfaces to reduce non-specific (usually hydrophobic) interactions from biomolecules in complex fluids. These treatments minimize adsorption of low concentrations of solutes to glass surfaces in chemistry and biology, but are not sufficient on their own to completely prevent blood coagulation or molecular adsorption.

Fluorous surfaces, i.e., surfaces that are treated to contain fluorocarbon moieties, have been used to form microarray surfaces. For example, glass slides having perfluorocarbon domains attached to molecules of interest have been used to immobilize molecules of interest on a surface.

Moreover, fluorous-treated microelectromechanical "MEM" devices have been used to address a well-known problem in the fabrication of MEM-stiction, which occurs when surface adhesion forces are higher than the mechanical restoring force of the micro-structure. One proposed solution has been to coat the MEMS surface with monomolecular coatings that are "Teflon-like" and covalently bonded to the MEMS surface. This approach fails to prevent adhesion of liquids.

The use of polymeric species to minimize protein adsorption and control blood clotting is known in the art. *See*, *e.g*., Barstad, R.M, et al., Thrombosis and haemostasis 79, 302-305 (1998); Niimi, Y., et al., Anesth. Analg. 89, 573-579 (1999); Chen, S. et al., Polymer 51, 5283-5293 (2010). However, such methods are not entirely effective at repelling blood and preventing blood clot formation without the use of anticoagulants. For example, heparinized polymer-coated products are known in the art However, because heparinized polymer-coated products are not sufficient to prevent blood clot adhesion, soluble heparin or anticoagulants must be added to the blood to fully prevent coagulation on devices.

EP 0 166 998 A2 discloses a medical instrument which exhibits low frictional resistance upon insertion when wetted with aqueous solution, for example, humor such as saliva, digestive fluid, blood, etc., physiological saline or water, having excellent retention and shelf stability, wherein a reactive functional group is covalently bonded to the surface of the medical instrument with a water-soluble polymer or derivative thereof.

An article entitled "Bioinspired self-repairing slippery surfaces with pressure-stable omniphobicity" authored by Tak-Sing Wong; Sung Hoon Kang; Sindy K. Y. Tang; Elizabeth J. Smythe; Benjamin D. Hatton; Alison Grinthal; Joanna Aizenberg; Nature (2011), 477, 443M47, discloses a self-healing, slippery liquid-infused porous surface(s) (SLIPS) with exceptional liquid- and ice-repellency, pressure stability and enhanced optical transparency. The approach in this prior art document was inspired by Nepenthes pitcher plants and uses nano/microstructured substrates to lock in place the infused lubricating fluid. The structured surfaces are capable of repelling various simple and complex liquids (water, hydrocarbons, crude oil and blood), maintaining low contact angle hysteresis (<2.5°) and quickly restoring liquid-repellency after physical damage (within 0.1-1 s).

### SUMMARY

There is a need for a repellant surface that prevents molecular or particulate adhesion to, fouling, and blood coagulation on a variety of commercially available surfaces.

According to the first aspect, a medical device having a slippery surface according to claim 1 is disclosed. The medical device (article) comprises a smooth substrate having a monomolecular fluorous anchoring layer. The anchoring layer comprises a head group that is covalently attached to the substrate and a functional group, which is directly or indirectly attached to the head group. The article also has a lubricating layer that comprises a lubricating liquid comprising a perfluorocarbon oil, which has an affinity for the functional group. The lubricating layer is disposed over the anchoring layer, and the layers are held together by non-covalent attractive forces. The anchoring layer and the lubricating layer form a slippery surface configured and arranged for contact with a material that is immiscible with the lubricating liquid. The immiscible material that is contacted to the thus formed slippery surface is repelled.

According to the second aspect, a method of preventing adhesion, adsorption, surface-mediated clot formation, or coagulation of a material on said medical device in accordance with claim 14 is disclosed.

In one or more embodiments, the material being repelled is selected from the group consisting of a liquid, solution, suspension, complex fluid, and a solid.

In one or more embodiments, the slippery surface is hydrophobic or even superhydrophobic. In other embodiments, the slippery surface is oleophobic or even superoleophobic. In still other embodiments, the slippery surface is omniphobic or even superomniphobic. In some embodiments, the surface could be amphiphilic.

In some embodiments, the functional group is a hydrocarbon.

In one or more embodiments, the functional group is a polar group, and the polar group optionally is selected from the group consisting of charged polypeptides, polyanions, polycations, polar polymers, polysaccharides, amines, carboxylic acids, guanidine, alcohols, sulfhydryls, carboxamides, metal oxides, inorganic oxides, and combinations thereof.

In one or more embodiments, the functional group is a perfluorocarbon. In one or more embodiments, the functional group is a partially fluorinated hydrocarbon.

In some embodiments, the functional group could consist of perfluoropolyethers, polyethers, polysulfides, polyolefins, polyesters, polyamides, polyamphiphiles, and polyampholytes as well as oligomeric forms of aforementioned polymers. An exemplar polyether is poly(propylene oxide) and an oligomeric polyether could be oligo(arylene ether sulfone) while an example of a polyolefin includes polypropylene.

In certain embodients, the head groups of the anchoring layer includes ethers, silyl ethers, siloxanes, esters of carboxylic acids, esters of sulfonic acids, esters of sulfinic acids, esters of sulfuric acids, esters of phosphonic acids, esters of phosphinic acids, esters of phosphoric acids, silyl esters of carboxylic acids, silyl esters of sulfonic acids, silyl esters of sulfinic acids, silyl esters of sulfuric acids, silyl esters of phosphonic acids, silyl esters of phosphinic acids, silyl esters of phosphoric acids, oxides, sulfides, carbocycles, heterocycles with at least one oxygen atom, heterocycles with at least one nitrogen atom, heterocycles with at least one sulfur atom, heterocycles with at least one silicon atom, 'click' reactions-derived heterocycles, Diels-Alder reactions-derived carbocycles, Diels-Alder reactions-derived heterocycles, amides, imides, sulfides, thiolates, metal thiolates, urethanes, oximes, hydrazides, hydrazones, physisorbed or chemisorbed or otherwise non-covalently attached moieties, or combinations thereof.

In certain embodiments, head groups include maleimides, acrylates, acrylamides, epoxides, aziridines, thiiranes, aldehydes, ketones, azides, alkynes, disulfides, anhydrides, carboxylates phosphates, phosphonates, sulfates, sulfonates, nitrates, amidine, silanes, siloxanes, cyanates, acetylenes, cyanides, halogens, acetals, ketals, biotin, cyclodextrins, adamantanes, and vinyls.

In one or more embodiments, the head group is silane, carboxylate, sulfonate or phosphonate.

In one or more embodiments, the surface is selected from the group consisting of acrylic, glass, polymers, metals, carbon, plastics, paper, ceramics, and combinations thereof.

In certain embodiments, the surface may be selected from biocompatible materials such as hydrogels, biopolymers, and polyesters.

In specific embodiments, the surface is selected from the group consisting of poly(dimethyl siloxane) (PDMS), acrylic, polystyrene, tissue-culture polystyrene, metal, polypropylene, acrylic adhesive, silicon wafer, polysulfone, and soda lime glass, the anchoring layer comprises a silyl group covalently attached to a perfluorocarbon tail, and the lubricating layer comprises perfluorocarbon oil. The anchoring layer and the lubricating layer can form an omniphobic slippery surface that repels an immiscible material.

In one or more embodiments, the surface is treated to activate the surface prior to exposure to the anchoring layer. In one or more aspects, activation comprises acid or base treatment, oxidization, ammonization, plasma, and microwave treatment (or combinations thereof). Activation of surfaces also may be carried out through chemical deposition (vapor or solution), aminolysis, acrylation, transesterification, reduction, nucleophilic or electrophilic substitution, ozonolysis, or irradiation to install reactive or functional groups for subsequent modification with an anchoring layer.

In one or more embodiments, the surface prevents coagulation of blood. In one or more embodiments, the substrate has micropassages through which lubricating liquid is replenished. In other embodiments, the substrate comprises a reservoir through which lubricating liquid is replenished. In still other embodiments, the substrate is a tubing through which boluses of lubricating liquid pass.

In any of the preceding embodiments, the immiscible material is a solid, which can be a particulate, including a dry particulate, or a solid surface. In other embodiments, the immiscible material is a liquid selected from the group consisting of viscous liquids, non-viscous, semi-solids, tacky liquids, and complex fluids. In other embodiments, the immiscible material is a dissolved molecule.

In one or more embodiments, the immiscible material contains an additive, which is selected from the group consisting of a solute, a particulate or a combination thereof. In one embodiment, the immiscible material is repelled by the surface and the additive is attracted to the surface. In another embodiment, both the immiscible material and the additive are repelled by the surface.

In one or more embodiments, the immiscible material is a bodily fluid. The bodily fluid may be selected from the group consisting of whole blood, plasma, serum, sweat, feces, urine, saliva, tears, vaginal fluid, prostatic fluid, gingival fluid, amniotic fluid, intraocular fluid, cerebrospinal fluid, seminal fluid, sputum, ascites fluid, pus, nasopharengal fluid, wound exudate fluid, aqueous humour, vitreous humour, bile, cerumen, endolymph, perilymph, gastric juice, mucus, peritoneal fluid, pleural fluid, sebum, vomit, and combinations thereof.

In one or more embodiments, the immiscible material is a solution or suspension containing bacteria. The bacteria may be selected from the group consisting of *Actinobacillus*, *Acinetobacter* (*e.g*., *Acinetobacter baumannii*), Aeromonas, *Bordetella*, *Brevibacillus*, *Brucella*, *Bacteroides*, *Burkholderia*, *Borelia*, *Bacillus*, *Campylobacter*, *Capnocytophaga*, *Cardiobacterium*, *Citrobacter*, *Clostridium*, *Chlamydia*, *Eikenella*, *Enterobacter*, *Escherichia*, *Francisella*, *Fusobacterium*, *Flavobacterium*, *Haemophilus*, *Helicobacter*, *Kingella*, *Klebsiella*, *Legionella*, *Listeria*, *Leptospirae*, *Moraxella*, *Morganella*, *Mycoplasma*, *Mycobacterium*, *Neisseria*, *Pasteurella*, *Proteus*, *Prevotella*, *Plesiomonas*, *Pseudomonas*, *Providencia*, *Rickettsia*, *Stenotrophomonas*, *Staphylococcus*, *Streptococcus* (group A), *Streptococcus agalactiae* (group B), *Streptococcus bovis*, *Streptococcus pneumoniae*, *Streptomyces*, *Salmonella*, *Serratia*, *Shigella*, *Spirillum*, *Treponema*, *Veillonella*, *Vibrio*, *Yersinia*, *Xanthomonas*, and combinations thereof. The slippery surface according to one or more embodiments prevents adhesion, growth or bio-fouling by the bacteria.

In one or more embodiments, the immiscible material is a solution or suspension containing fungi. The fungus may be selected from the group consisting of a member of the genus *Aspergillus*, *Blastomyces dermatitidis*, *Candida*, *Coccidioides immitis*, *Cryptococcus*, *Histoplasma capsulatum* var. *capsulatum*, *Histoplasma capsulatum* var. *duboisii*, *Paracoccidioides brasiliensis*, *Sporothrix schenckii*, *Absidia corymbifera*; *Rhizomucor pusillus*, *Rhizopus arrhizous*, and combinations thereof. The slippery surface according to one or more embodiments prevents adhesion, growth or bio-fouling by the fungus.

In one or more embodiments, the material is a solution or suspension containing virus. The virus may be selected from the group consisting of cytomegalovirus (CMV), dengue, Epstein-Barr, Hantavirus, human T-cell lymphotropic virus (HTLV I/II), Parvovirus, hepatitides, human papillomavirus (HPV), human immunodeficiency virus (HIV), acquired immunodeficiency syndrome (AIDS), respiratory syncytial virus (RSV), Varicella zoster, West Nile, herpes, polio, smallpox, yellow fever, rhinovirus, coronavirus, Orthomyxoviridae (influenza viruses), and combinations thereof. The slippery surface according to one or more embodiments prevents adhesion or growth by the virus.

In one or more embodiments, the material is a solution or suspension containing particles selected from the group consisting of normal cells, diseased cells, parasitized cells, cancer cells, foreign cells, stem cells, and infected cells, microorganisms, viruses, virus-like particles, bacteria, bacteriophages, proteins, cellular components, cell organelles, cell fragments, cell membranes, cell membrane fragments, viruses, virus-like particles, bacteriophage, cytosolic proteins, secreted proteins, signaling molecules, embedded proteins, nucleic acid/protein complexes, nucleic acid precipitants, chromosomes, nuclei, mitochondria, chloroplasts, flagella, biominerals, protein complexes, and minicells.

In yet another aspect, an article having a slippery surface includes a sterile substrate comprising an anchoring layer, the anchoring layer comprising a head group attached to the substrate and a functional group directly or indirectly attached to the head group and a lubricating layer comprising a lubricating liquid having an affinity for the functional group and disposed over the anchoring layer, wherein the anchoring layer and the lubricating layer are held together by non-covalent attractive forces, wherein the anchoring layer and the lubricating layer form a slippery surface configured and arranged for contact with a material that is immiscible with the lubricating liquid.

In one or more embodiments, the substrate is silanized before sterilization.

### BRIEF DESCRIPTION OF THE DRAWINGS

The following figures are provided for the purpose of illustration only and are not intended to be limiting.

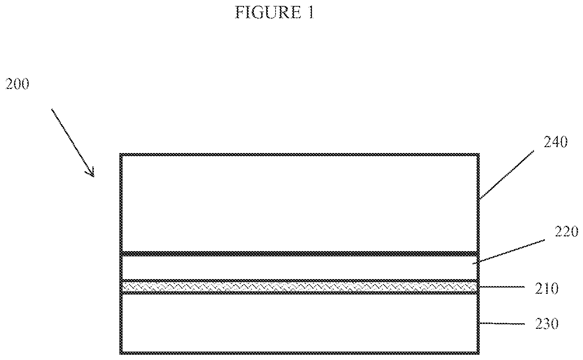

**FIG. 1** is a schematic of a slippery liquid immobilized coating surface in accordance with the present disclosure, in which an anchoring layer of immobilized molecules that exhibit chemical properties necessary to interact with and retain a lubricating layer of a lubricating liquid molecules that are immiscible with a repellent material.

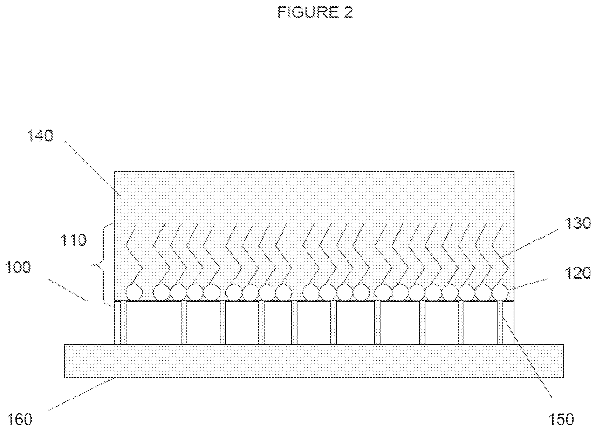

**FIG. 2** is a schematic illustration of an ultra-slippery surface with a reservoir, which replenishes the surface with lubricating liquid, housed below the repellant surface, according to one or more embodiments.

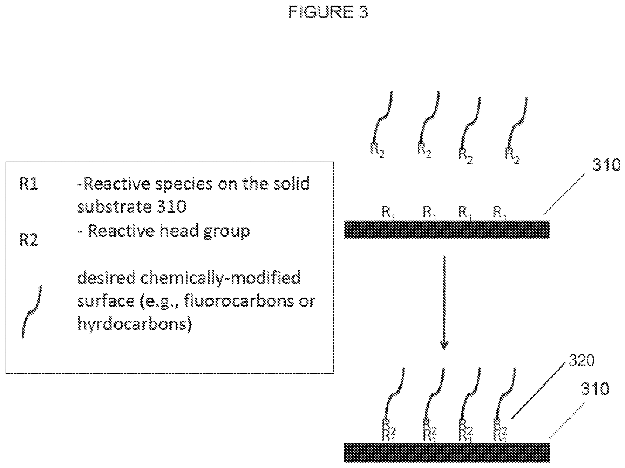

**FIG. 3** is a schematic of the generic cross-linking chemistry used to adjust the affinity of the surface to the liquid(s) to which it will be exposed, in which R1 represents the reactive moieties of the surface of the substrate, and R2 represents the reactive moieties on the bifunctional molecules used to modify the surface.

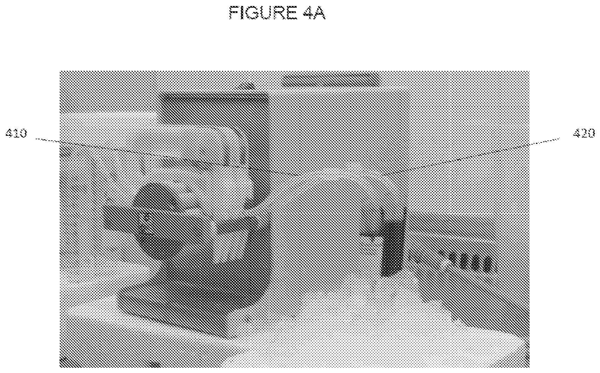

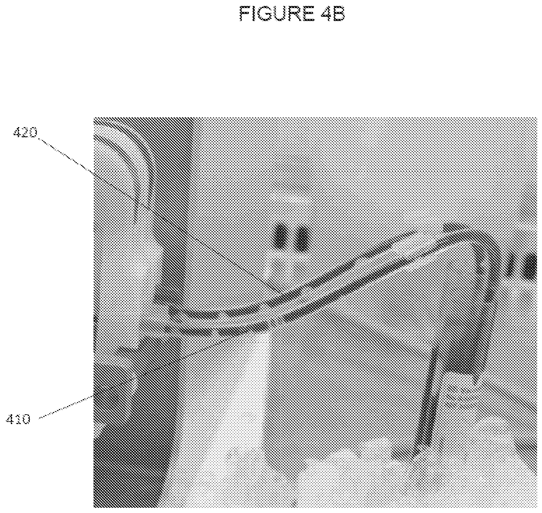

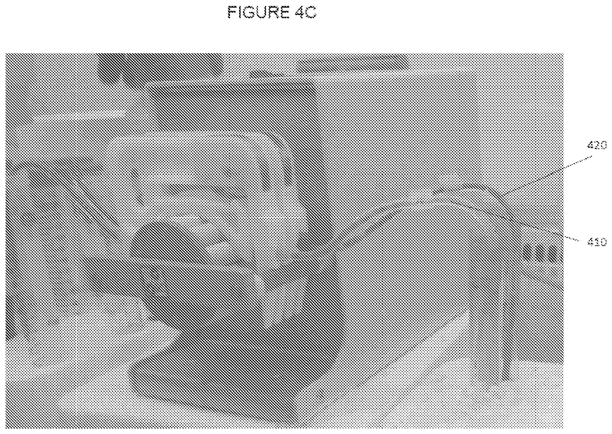

**FIG. 4** is a series of images of PDMS tubing through which a plug of PFC oil is pumped through silanized and unsilanized tubing (**FIG. 4A**), followed by ice-chilled untreated human blood demonstrating that the combination of silanized PDMS tubing with an overlaid coating of PFC oil successfully prevented adhesion of untreated human blood on ice for 3 minutes (**FIG. 4B**) and 45 minutes (**FIG. 4C**). Clear droplets in figures are PFC oil.

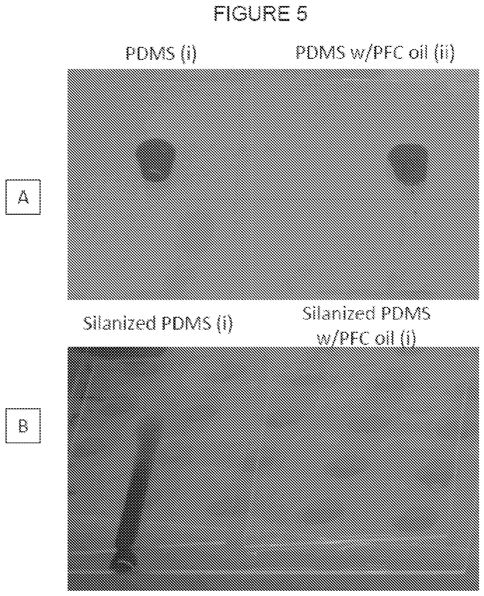

**FIG. 5** is a series of images showing fresh human blood without anti-coagulants repelled by an ultra-slippery surface composed of a silanized PDMS surface coated with a thin liquid layer of PFC oil (tridecafluorotetrahydrooctyltrichlorosilane) (**FIG. 5B(ii)**), but adhering to an untreated and unmodified PDMS surface (**FIG. 5A(i)**), non-silanized PDMS with PFC oil (**FIG. 5A(ii)**), and silanized PDMS without a liquid oil layer (**FIG. 5B(i)**).

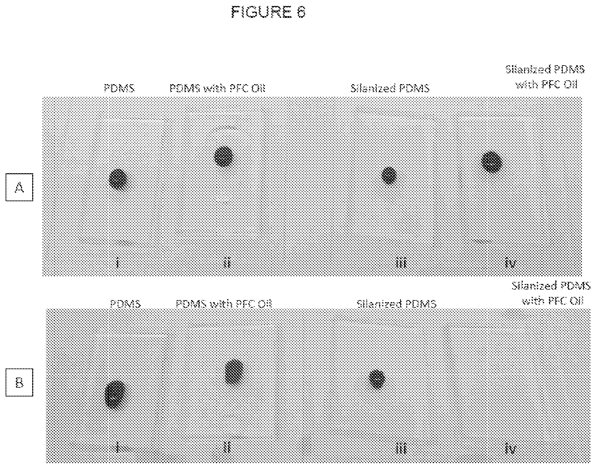

**FIG. 6** shows a series of images in which a drop of blood has been applied to PDMS sheets having various surface treatments before (**FIG. 6A**) and after (**FIG. 6B**) tilting, demonstrating that blood adhered to untreated and unmodified PDMS (**FIG. 6B(i)**), PDMS with PFC oil (trichloro(1H,1H,2H,2H-perfluorooctyl)silane) (**FIG. 6B(ii)**), and silanized PDMS (**FIG. 6B(iii)**), but was completely repelled by silanized PDMS coated with a thin PFC oil layer (**FIG. 6B(iv)**).

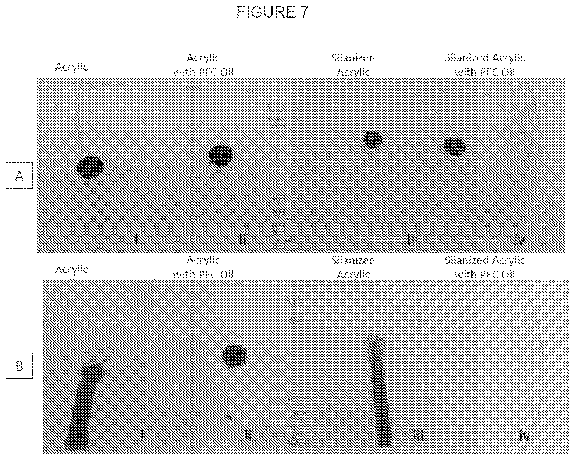

**FIG. 7** shows a series of images in which a drop of blood has been applied to acrylic sheets having various surface treatments before (**FIG. 7A**) and after (**FIG. 7B**) tilting, demonstrating that blood adhered to untreated and unmodified acrylic (**FIG. 7B(i)**), acrylic with PFC oil (**FIG. 7B(ii)**), and silanized acrylic (**FIG. 7B(iii)**), but was completely repelled by silanized acrylic coated with a thin PFC oil layer (**FIG. 7B****(iv)**).

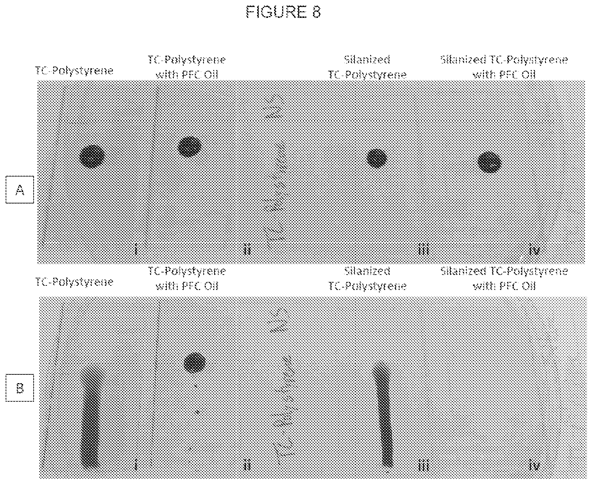

**FIG. 8** shows a series of images in which a drop of blood has been applied to tissue-culture polystyrene sheets having various surface treatments before (**FIG. 8A**) and after (**FIG. 8B**) tilting, demonstrating that blood adhered to untreated and unmodified tissue-culture polystyrene (**FIG. 8B(i)**), tissue-culture polystyrene with PFC oil (**FIG. 8B(ii)**), and silanized tissue-culture polystyrene (**FIG. 8B(iii)**), but was completely repelled by silanized tissue-culture polystyrene with a PFC oil coating (**FIG. 8B****(iv)**).

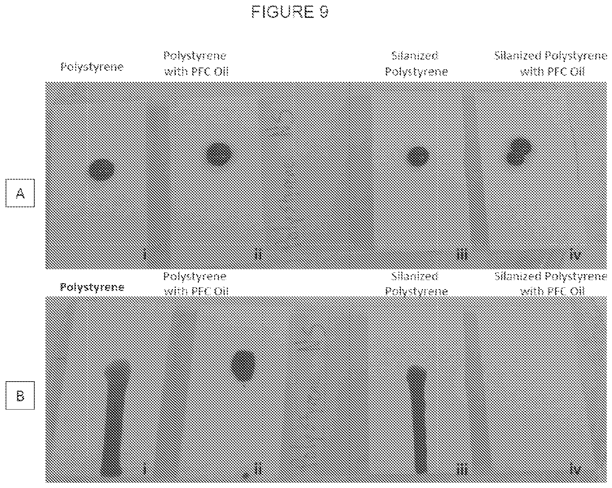

**FIG. 9** shows a series of images in which a drop of blood has been applied to polystyrene sheets having various surface treatments before (**FIG. 9A**) and after (**FIG. 9B**) tilting, demonstrating that blood adhered to untreated and unmodified polystyrene (**FIG. 9B(i)**), polystyrene with PFC oil (**FIG. 9B(ii)**), and silanized polystyrene (**FIG. 9B(iii)**), but was completely repelled by silanized polystyrene with a PFC oil coating (**FIG. 9B****(iv)**).

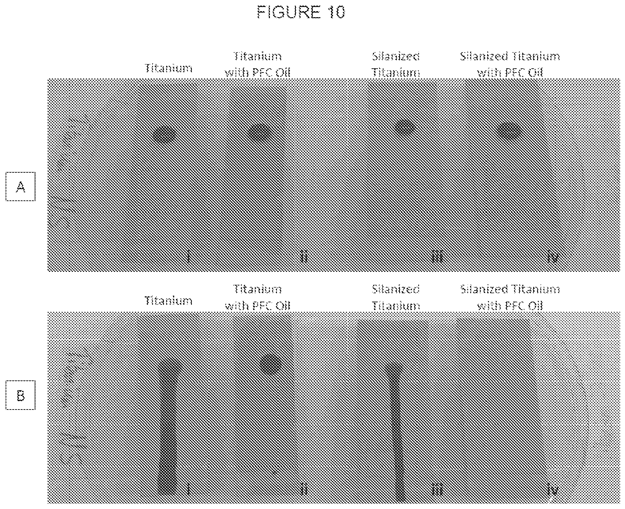

**FIG. 10** shows a series of images in which a drop of blood has been applied to titanium sheets having various surface treatments before (**FIG. 10A**) and after (**FIG. 10B**) tilting, demonstrating that blood adhered to untreated and unmodified titanium (**FIG. 10B(i)**), titanium with PFC oil (**FIG. 10B(ii)**), and silanized titanium (**FIG. 10B(iii)**), but was completely repelled by silanized titanium with a PFC oil coating (**FIG. 10B****(iv)**).

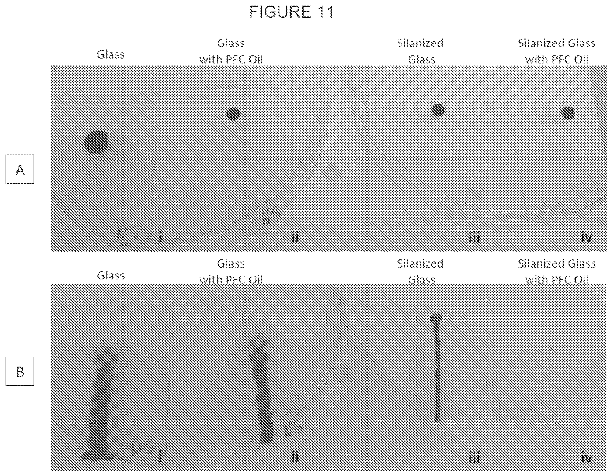

**FIG. 11** shows a series of images in which a drop of blood has been applied to soda lime glass having various surface treatments before (**FIG. 11A**) and after (**FIG. 11B**) tilting, demonstrating that blood adhered to untreated and unmodified soda lime glass (**FIG. 11B(i)**), soda lime glass with PFC oil (**FIG. 11B(ii)**), and silanized soda lime glass (**FIG. 11B(iii)**), but was completely repelled by silanized soda lime glass with a PFC oil coating (**FIG. 11B(iv)**).

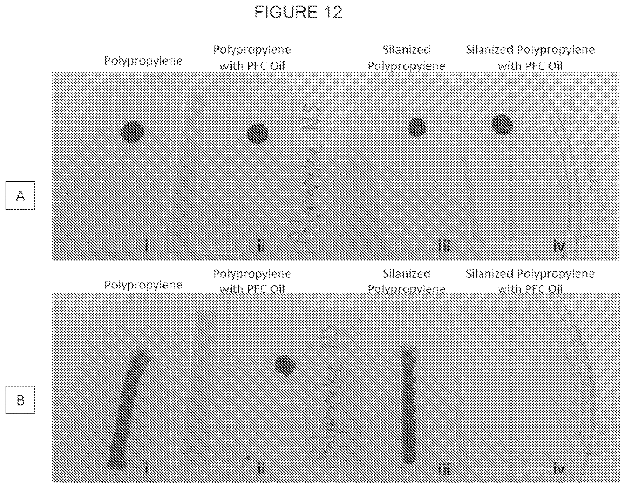

**FIG. 12** shows a series of images in which a drop of blood has been applied to polypropylene sheets having various surface treatments before (**FIG. 12A**) and after (**FIG. 12B**) tilting, demonstrating that blood adhered to untreated and unmodified polypropylene (**FIG. 12B(i)**), polypropylene with PFC oil (**FIG. 12B(ii)**), and silanized polypropylene with acrylic adhesive (**FIG. 12B(iii)**), but was completely repelled by silanized polypropylene with PFC oil coating (**FIG. 12B****(iv)**).

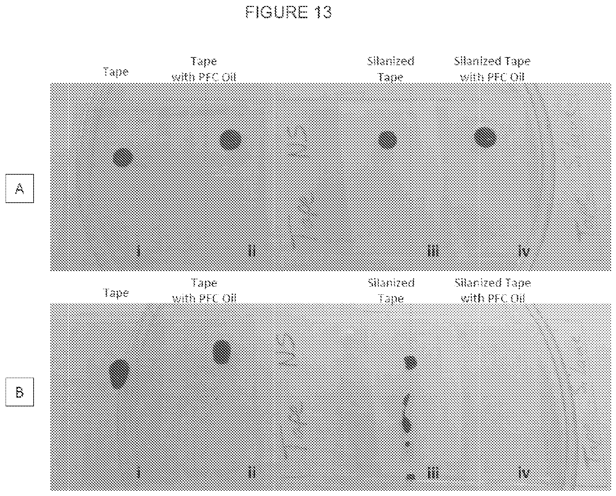

**FIG. 13** shows a series of images in which a drop of blood has been applied to polypropylene with acrylic adhesive sheets having various surface treatments before (**FIG. 13A**) and after (**FIG. 13B**) tilting, demonstrating that blood adhered to untreated and unmodified polypropylene with acrylic adhesive (**FIG. 13B(i)**), polypropylene with acrylic adhesive with PFC oil (**FIG. 13B(ii)**), and silanized polypropylene with acrylic adhesive (**FIG. 13B(iii)**), but was completely repelled by silanized polypropylene with acrylic adhesive with a PFC oil coating (**FIG. 13B****(iv)**).

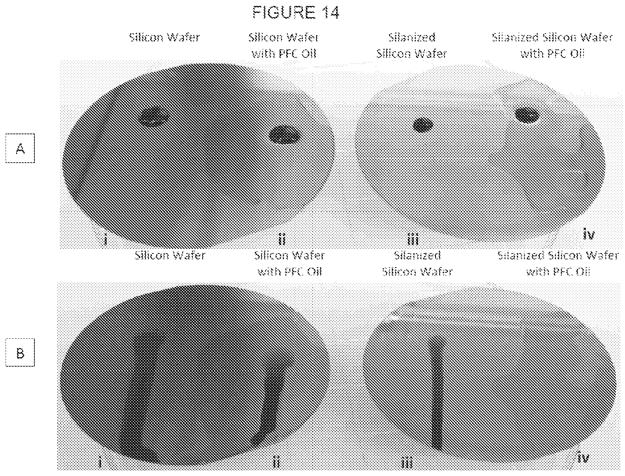

**FIG. 14** shows a series of images in which a drop of blood has been applied to silicon wafers having various surface treatments before (**FIG. 14A**) and after (**FIG. 14B**) tilting, demonstrating that blood adhered to untreated and unmodified silicon wafer (**FIG. 14B(i)**), silicon wafer with PFC oil (**FIG. 14B(ii)**), and silanized silicon wafer (**FIG. 14B(iii)**), but was completely repelled by silanized silicon wafer with a PFC oil coating (**FIG. 14B****(iv)**).

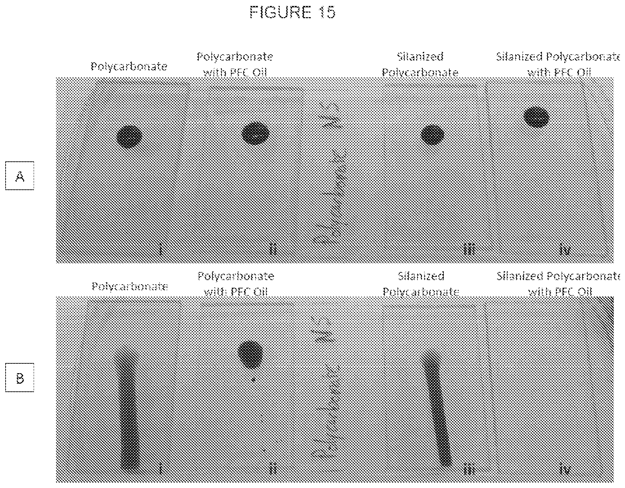

**FIG. 15** shows a series of images in which a drop of blood has been applied to polycarbonate sheets having various surface treatments before (**FIG. 15A**) and after (**FIG. 15B**) tilting, demonstrating that blood adhered to untreated and unmodified polycarbonate (**FIG. 15B(i)**), polycarbonate with PFC oil (**FIG. 15B(ii)**), and silanized polycarbonate (**FIG. 15B(iii)**), but was completely repelled by silanized polycarbonate with a PFC oil coating (**FIG. 15B****(iv)**).

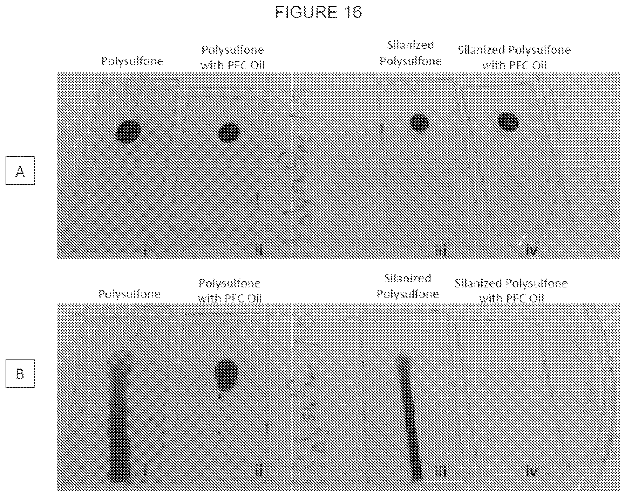

**FIG. 16** shows a series of images in which a drop of blood has been applied to polysulfone sheets having various surface treatments before (**FIG. 16A**) and after (**FIG. 16B**) tilting, demonstrating that blood adhered to untreated and unmodified polysulfone (**FIG. 16B(i)**), polysulfone with PFC oil (**FIG. 16B(ii)**), and silanized polysulfone (**FIG. 16B(iii)**), but was completely repelled by silanized polysulfone with a PFC oil coating (**FIG. 16B****(iv)**).

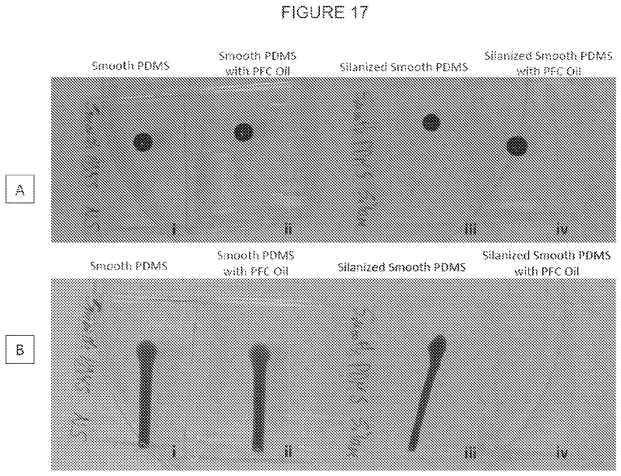

**FIG. 17** shows a series of images in which drops of blood have been applied to smooth PDMS sheets, where the PDMS was cured on a stainless steel surface having an average roughness of 0.1 micrometers, having various surface treatments before (**FIG. 17A**) and after (**FIG. 17B**) tilting, demonstrating that blood adhered to untreated and unmodified PDMS (**FIG. 17B(i)**), PDMS with PFC oil (**FIG. 17B(ii)**), and silanized PDMS (**FIG. 17B(iii)**), but was completely repelled by silanized PDMS with a PFC oil coating (**FIG. 17B****(iv)**).

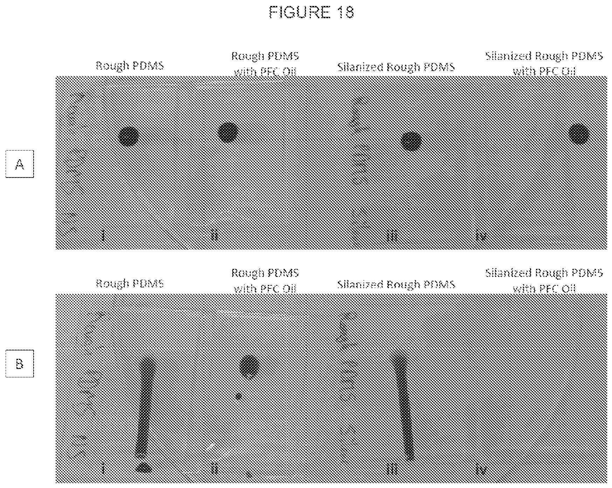

**FIG. 18** shows a series of images in which drops of blood have been applied to rough PDMS sheets, where the PDMS was cured on a stainless steel surface having an average roughness of 1.0 micrometers, having various surface treatments before (**FIG. 18A**) and after (**FIG. 18B**) tilting, demonstrating that blood adhered to rough, untreated and unmodified PDMS (**FIG. 18B(i)**), rough PDMS with PFC oil (**FIG. 18B(ii)**), and rough, silanized PDMS (**FIG. 18B(iii)**), but was completely repelled by rough, silanized PDMS with a PFC oil coating (**FIG. 18B****(iv)**).

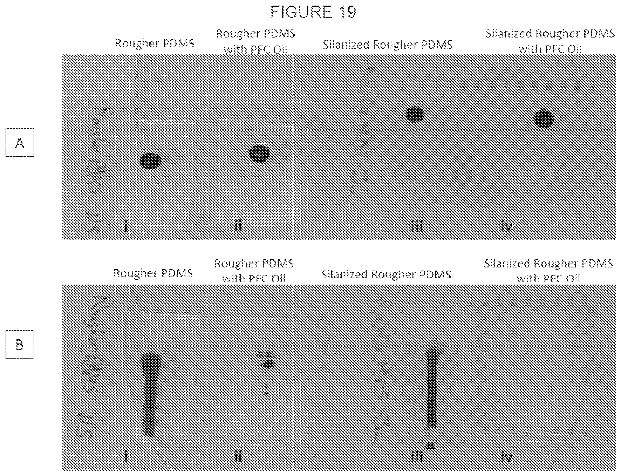

**FIG. 19** shows a series of images in which drops of blood have been applied to PDMS sheets, where the PDMS was cured on a stainless steel surface having an average roughness of 2.0 micrometers, having various surface treatments before (**FIG. 19A**) and after (**FIG. 19B**) tilting, demonstrating that blood adhered to rougher, untreated and unmodified PDMS (**FIG. 19B(i)**), rougher PDMS with PFC oil (**FIG. 19B(ii)**), and rougher, silanized PDMS (**FIG. 19B(iii)**), but was completely repelled by rougher, silanized PDMS with a PFC oil coating (**FIG. 19B****(iv)**).

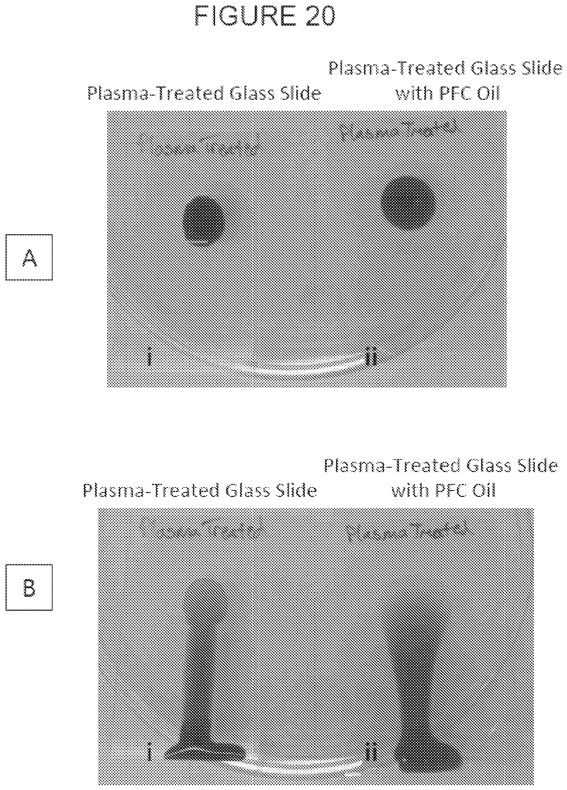

**FIG. 20** shows two slides of plasma-treated soda lime glass, each containing a droplet of anticoagulant-free blood, in the untilted (**FIG. 20A**) and tilted (**FIG. 20B**) state.

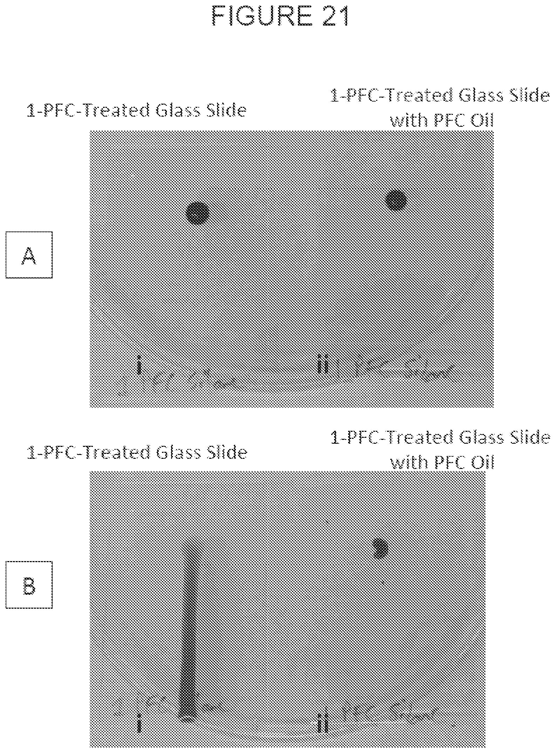

**FIG. 21** shows images of plasma-treated, trifluoropropyltrichlorosilane-treated soda lime glass slides, silanized with a molecule that has a fluoridated carbon tail one carbon long (1 -PFC) in the untilted (**FIG. 21A**) and untilted (**FIG. 21B**) state, demonstrating that blood adhered to the plasma-treated 1-PFC-treated glass side without PFC oil (**FIG. 21B(i)**), but much of the blood is repelled on the plasma-treated 1-PFC-treated glass slide with a PFC oil coating (**FIG. 21B(ii)**).

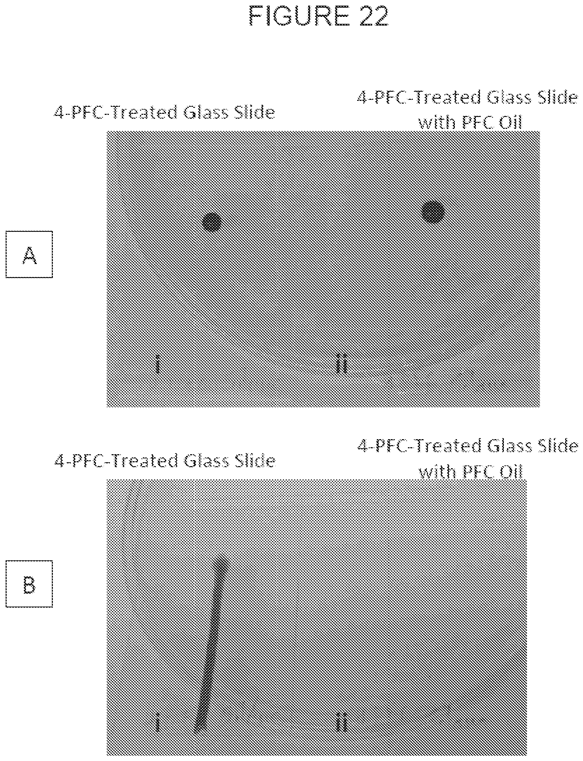

**FIG. 22** shows images of plasma-treated, nonafluorohexyltrichlorosilane-treated soda lime glass slides, silanized with a molecule that has a fluoridated carbon tail four carbons long (4-PFC) in the untilted (**FIG. 22A**) and untilted (**FIG. 22B**) state, demonstrating that blood adhered to the plasma-treated 4-PFC-treated glass side without PFC oil (**FIG. 22B(i)**), but all of the blood is repelled on the plasma-treated 4-PFC-treated glass slide with a PFC oil coating (**FIG. 22B(ii)**).

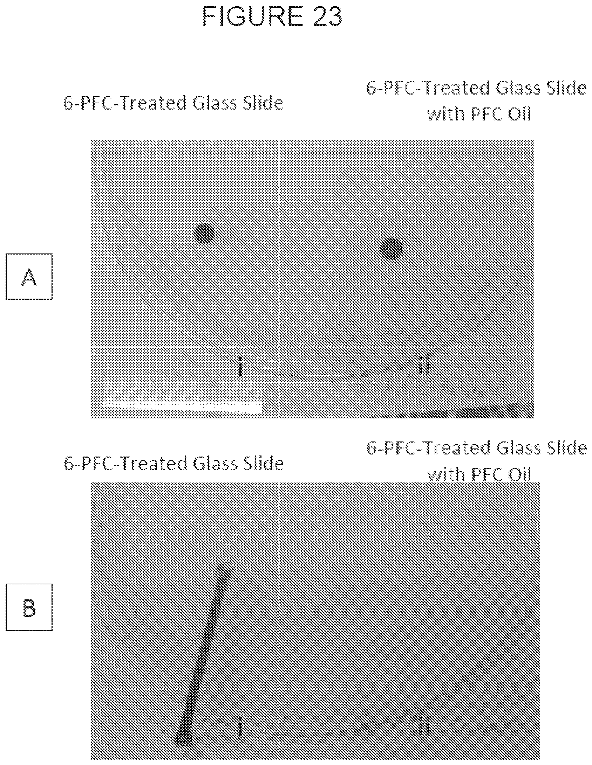

**FIG. 23** shows images of plasma-treated, tridecafluorotetrahydrooctyltrichlorosilane-treated soda lime glass slides, are silanized with a molecule that has a fluoridated carbon tail six carbons long (6-PFC) in the untilted (**FIG. 23A**) and untilted (**FIG. 23B**) state, demonstrating that blood adhered to the plasma-treated 6-PFC-treated glass side without PFC oil (**FIG. 23B(i)**), but all of the blood is repelled on the plasma-treated 6-PFC-treated glass slide with a PFC oil coating (**FIG. 23B(ii)**).

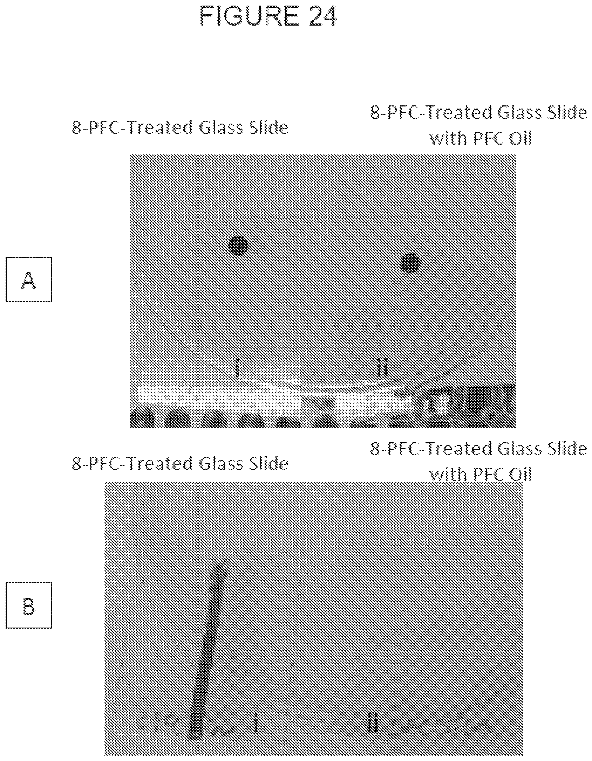

**FIG. 24** shows images of plasma-treated, heptadecafluorotetrahydrodecyltrichlorosilane-treated soda lime glass slides, silanized with a molecule that has a fluoridated carbon tail eight carbons long (8-PFC) in the untilted (**FIG. 24A**) and untilted (**FIG. 24B**) state, demonstrating that blood adhered to the plasma-treated 8-PFC-treated glass side without PFC oil (**FIG. 24B(i)**), but all of the blood is repelled on the plasma-treated 8-PFC-treated glass slide with a PFC oil coating (**FIG. 24B(ii)**).

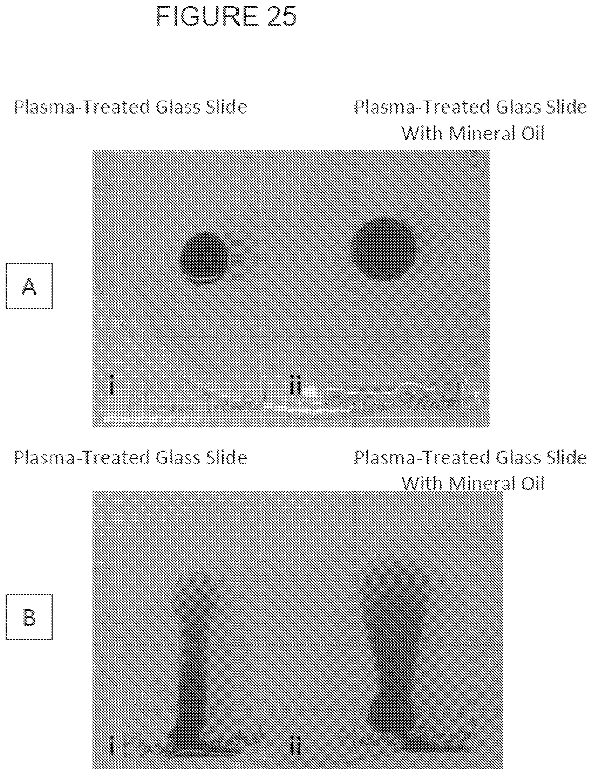

**FIG. 25** (not according to the present disclosure) shows a series of images of plasma-treated glass slides, for which mineral oil is applied to one slide (**FIG. 25A(ii)** and **FIG. 25B(ii)**), anticoagulant-free blood is pipetted onto both slides, and the slide are untilted (**FIG. 25A**) and tilted (**FIG. 25B**).

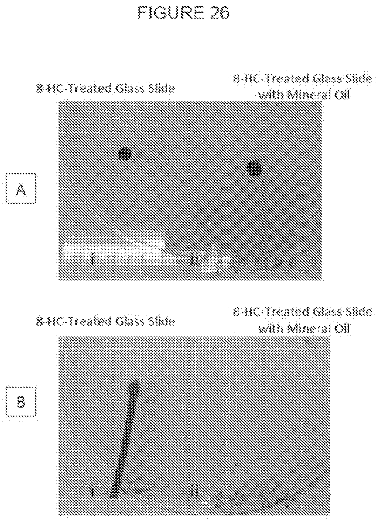

**FIG. 26** (not according to the present disclosure) shows a series of images of plasma-treated glass slides further modified with a silane with a linear octane (C8) tail. for which mineral oil is applied to one slide (**FIG. 26A(ii)** and **FIG. 26B(ii)**), anticoagulant-free blood is pipetted onto both slides, and the slides are untilted (**FIG. 26A**) and tilted (**FIG. 26B**)..

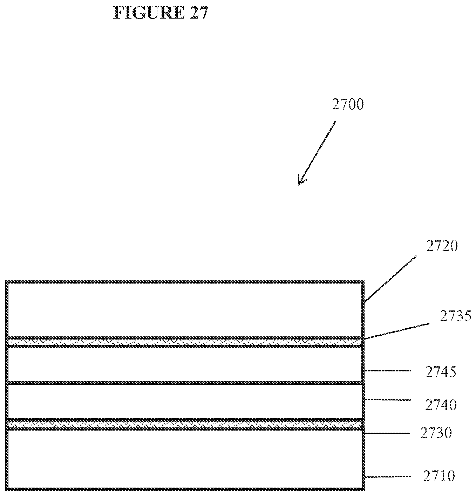

**FIG. 27** is a schematic illustration of slippery surface according to one or more embodiments useful in preventing two substrates from adhering to one another.

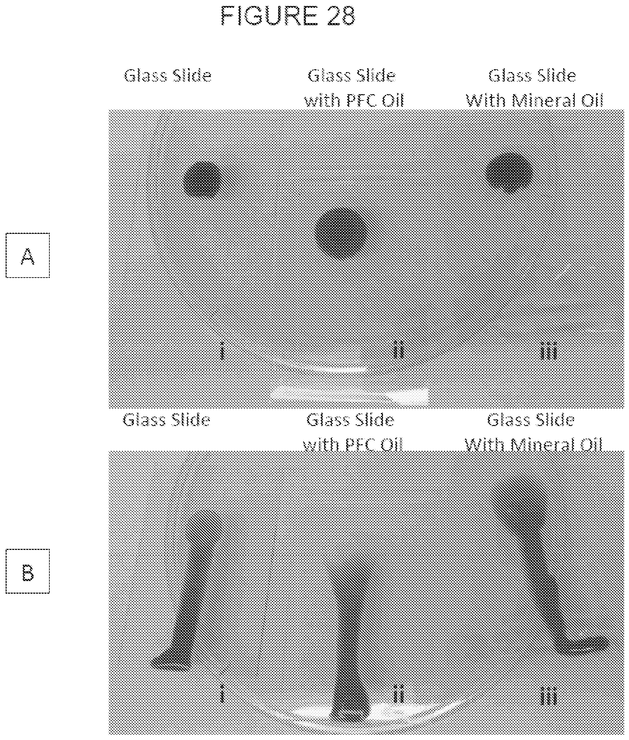

**FIG. 28** shows a series of images of glass slides that were not plasma treated, for which PFC oil is added to one slide (**FIG. 28A(ii)**) and mineral oil is applied to another slide (**FIG. 28B(iii)** and anticoagulant-free blood is pipetted onto each slide in the untilted (**FIG. 28A**) and tilted (**FIG. 28B**) state.

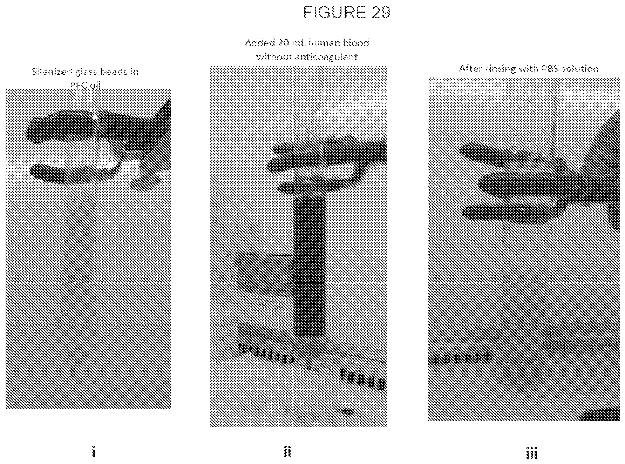

**FIG. 29** shows three images of 1 mm glass beads silanized in 5 % v/v in ethanol (nonafluorohexyltrichlorosilane, Gelest, SIN6597.6), and then (i) immersed in PFC oil (Fluorinert FC-70) to create an ultra-slippery surface on the beads; (ii) exposed to human blood without anticoagulant; and (iii) rinsed with PBS solution, demonstrating little to no adhesion of blood material.

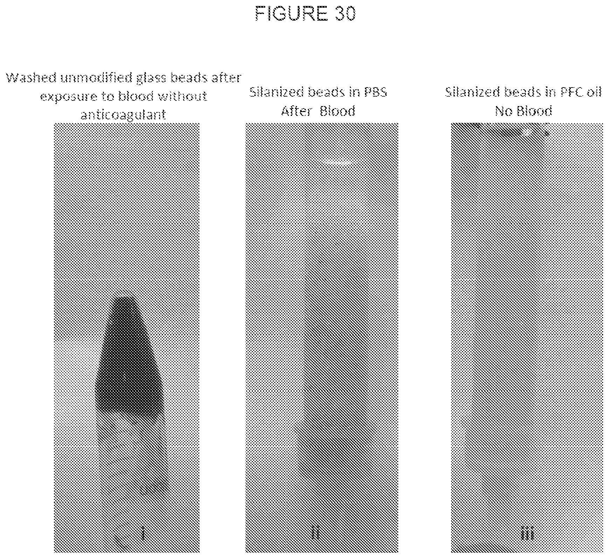

**FIG. 30** shows (i) washed, unmodified glass beads that have been exposed to anticoagulant-free blood, which forms a solid clot around the beads; (ii) silanized, PFC oil coated beads after blood had been pipetted onto the beads were washed with PBS and showed only minor amounts of adhesion of blood material on the beads; and (iii) silanized beads with a PFC oil coating before exposure to anticoagulant-free blood for comparison.

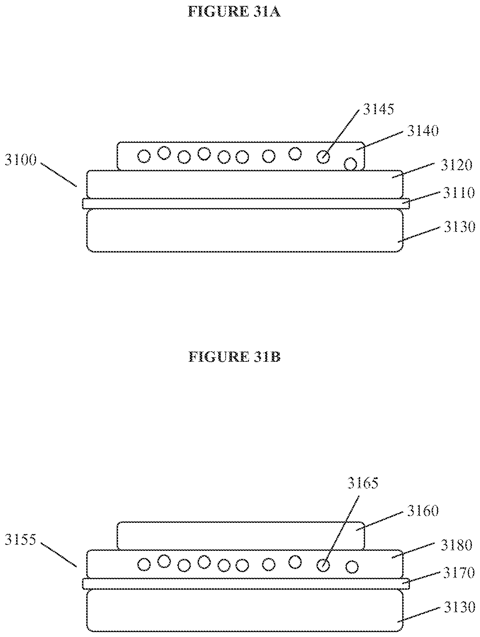

**FIG. 31A** is a schematic representation of an ultra-slippery surface that prevents repellent liquid, and solutes, solid particulates, or combinations thereof contained in the repellent liquid, from adhering to a substrate according to one or more embodiments.
**FIG. 31B** is a schematic representation of an ultra-slippery surface that prevents repellent liquid from adhering to a substrate, but allows the solutes, particulates, or combinations thereof to adhere to, or be retained on a substrate or in the lubricating liquid according to one or more embodiments.

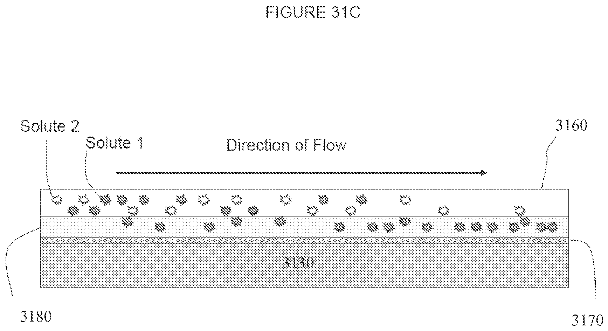

**FIG. 31C** is a schematic representation of an ultra-slippery surface that prevents repellent liquid from adhering to a substrate, but demonstrates selective affinity of the ultra slippery surface for certain solutes and/or particulates according to one or more embodiments .

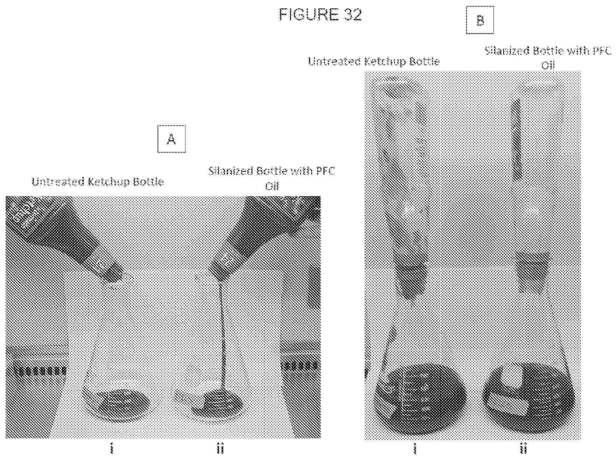

**FIG. 32 A-B** (not according to the present disclosure) is a series of images which shows that commercially-available plastic ketchup bottles made of PETE can be modified with silane and treated with PFC oil to create an ultra-slippery surface that prevents deposition of ketchup on the bottle walls.

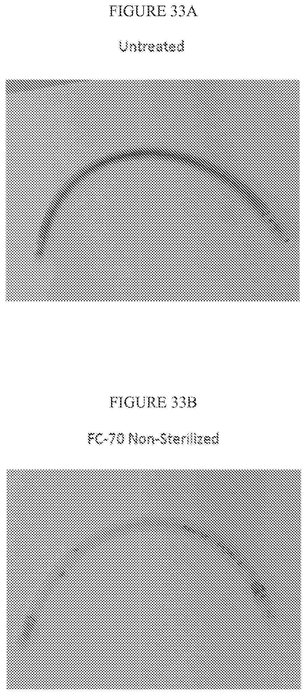

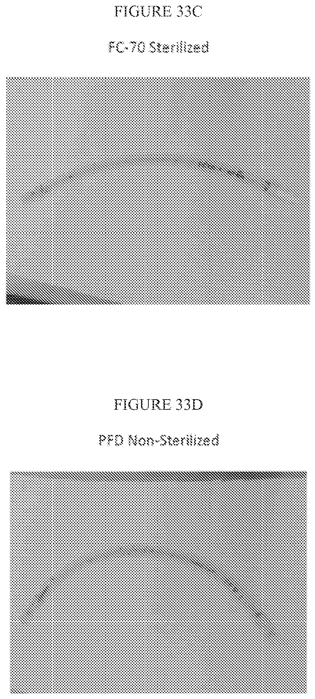

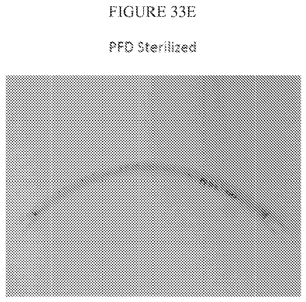

**FIGS. 33A-33E** are a series of photographs showing blood residue in untreated or treated medical grade PVC tubes after exposure to anticoagulant-free blood: (**FIG. 33A**) untreated tubes; (**FIG. 33B**) coated with FC-70, non-sterilized; (**FIG. 33C**) coated with FC-70, sterilized; (**FIG. 33D**) coated with PFD, non-sterilized; and (**FIG. 33E**) coated with PFD, sterilized.

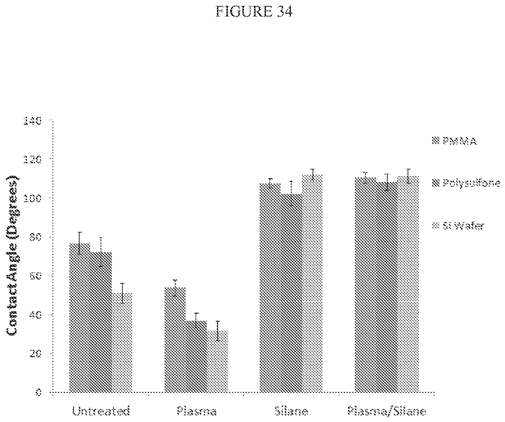

**FIG. 34** shows contact angle measurements carried out on PMMA, polysulfone and silicon wafer substrate before and after plasma treatment, silanization, and plasma treatment and silanization.

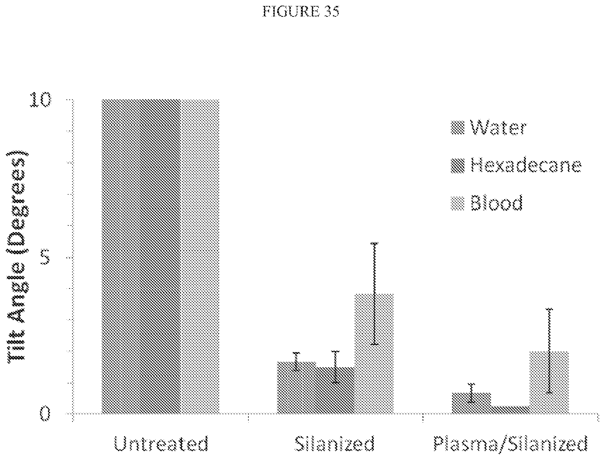

**FIG. 35** shows tilt angle measurements using water, hexadecane and blood, carried out on PMMA substrate before and after plasma treatment, silanization, and plasma treatment and silanization followed by a dip-coating in a lubricating liquid.

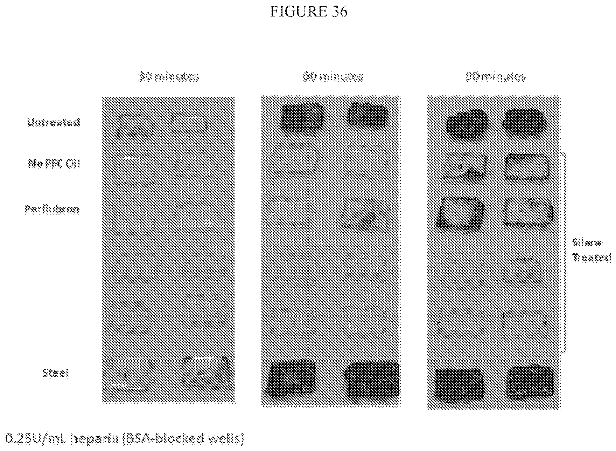

**FIG. 36** shows polysulfone surfaces exposed to human blood with and without various coated surfaces as well as a steel substrate.

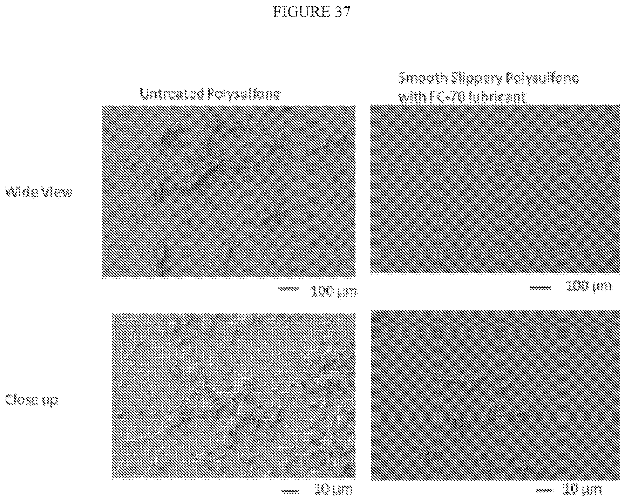

**FIG. 37** shows scanning electron microscope images of thrombus accumulation on untreated polysulfone surface and a FC-70 coated silanized polysulfone surface at wide and close up views.

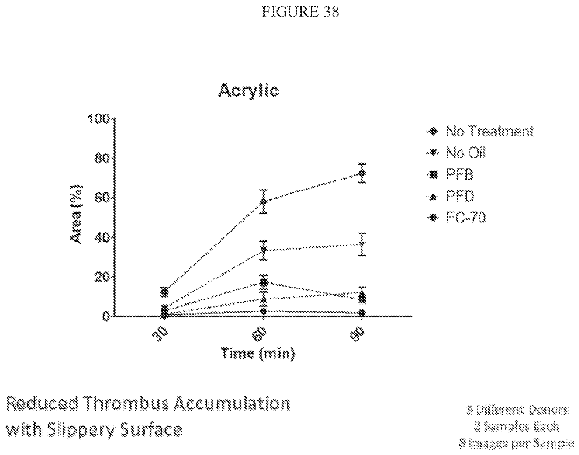

**FIG. 38** is a plot of percent fibrinogen coated area vs. time to demonstrate thrombus accumulation on PMMA substrate before and after formation of various different coated surfaces.

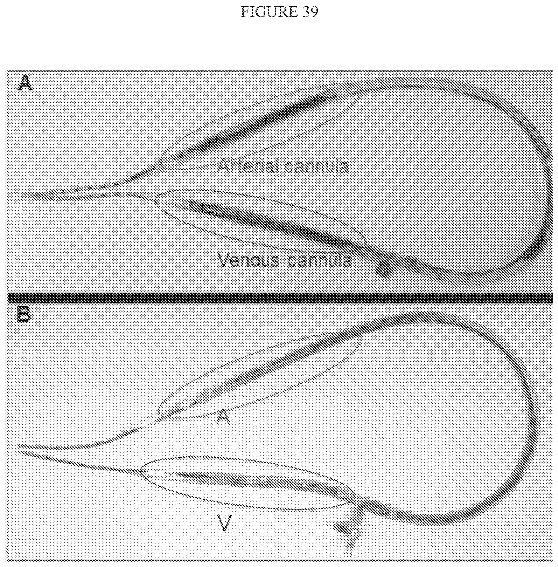

**FIG. 39 A-B** shows reduced thrombus accumulation in *in vivo* study carried out on PMMA substrate before and after formation of various different coated surfaces.

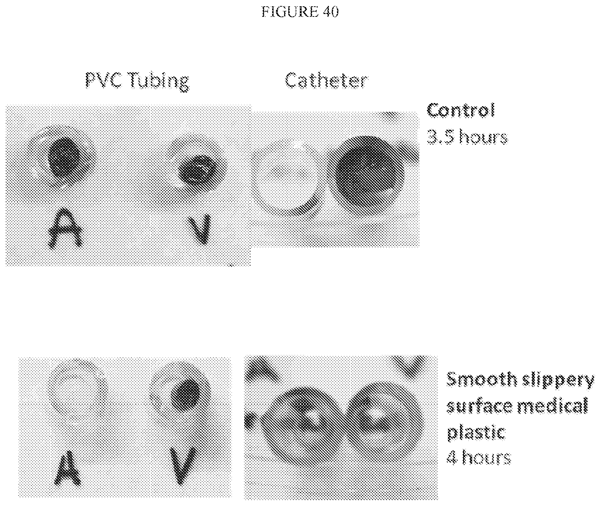

**FIG. 40** shows cross sectional images of untreated and coated PVC tubing and catheter demonstrating reduced thrombus accumulation in coated PVC tubing and catheter.

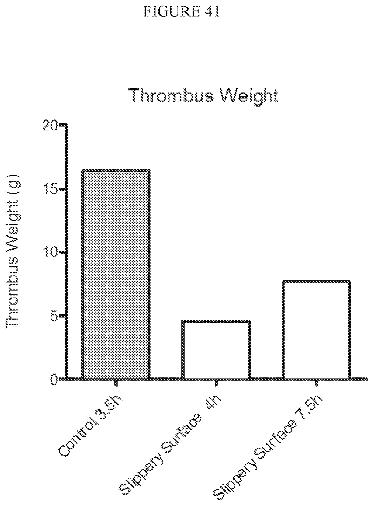

**FIG. 41** shows thrombus weight measurement of untreated and coated samples demonstrating reduced thrombus accumulation in coated samples.

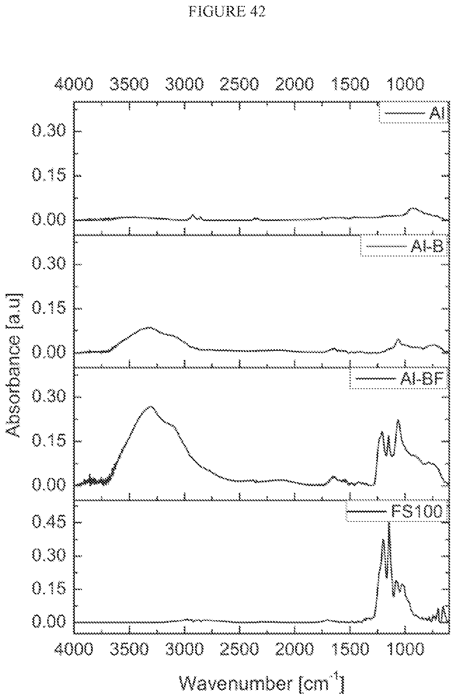

**FIG. 42** shows FT-IR spectra of bare aluminum (Al), aluminum oxy hydroxide (AlB), fluoro-functionalized aluminum oxy hydroxide (Al-BF), and pure fluoroaliphatic phosphate ester fluorosurfactant (FS100).

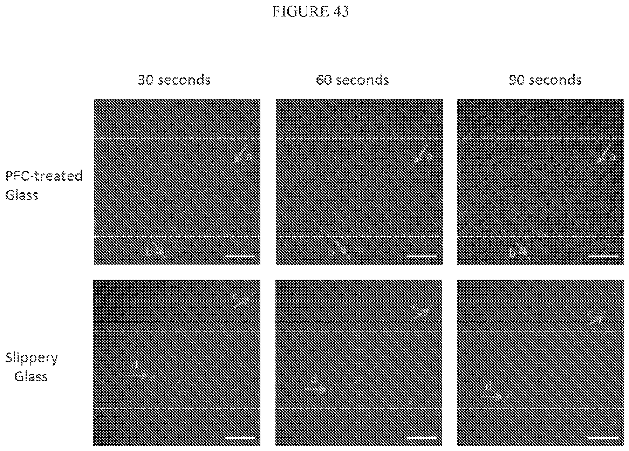

**FIG. 43** shows optical images of fibrinogen particles to glass without perfluorocarbon but do not stick to surfaces treated with perfluorocarbon.

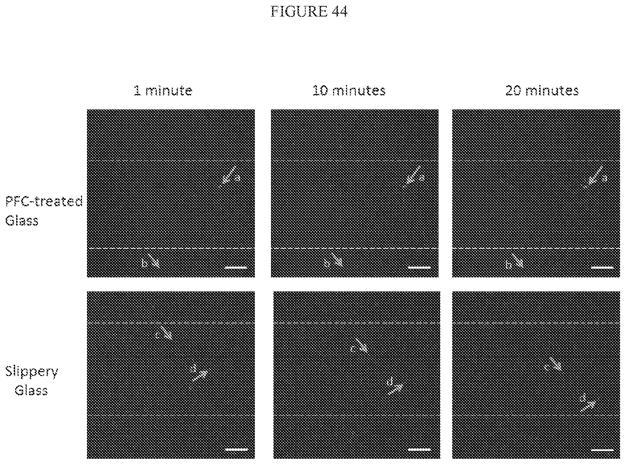

**FIG. 44** shows optical images of fluorescently labeled thrombi fibrinogen particles to glass without perfluorocarbon but do not stick to surfaces treated with perfluorocarbon.

### DETAILED DESCRIPTION

Methods for making medical device surfaces ultra-repellant to liquids, molecules or particulates contained within liquids, and dry solids are described. Further, methods for reducing adhesion and friction between two such surfaces are provided. The disclosed slippery liquid immobilized coating surfaces are synthetic surfaces that include an anchoring layer secured to an underlying substrate that interacts with and retains a thin layer of a lubricating liquid. The anchoring layer includes moieties having head groups that interact preferentially with the underlying surface and present functional groups to the environment that have surface properties that interact favorably with the lubricating liquid. The moieties are arranged on the underlying surface to form an immobilized molecular anchoring layer on the surface. The lubricating layer forms a monomolecular layer over the anchoring layer, and the anchoring layer and the lubricating layer are held together by non-covalent attractive forces.

Certain embodiments of the present invention relate generally to surface coatings of a polymeric, glass, metallic, metal oxide, or composite substrate by organic ligands and mixtures of organic ligands and the uses of such chemically modified substrates for forming slippery surfaces by infusing a liquid lubricant onto a chemically functionalized substrate.

Certain embodiments of the present invention provide compositions of organic ligand and methods of forming coated substrates that offer control of surface energy, affinity, and compatibility with applied liquid lubricant, and improved stability and retention of such lubricant on the functionalized substrates.

The chemically functionalized substrates are useful when forming ultrasmooth, omni-repellent, self-healing, anti-coagulating, and slippery surfaces by infusing lubricant onto the chemically functionalized surfaces. The compositions allow for tailoring of the type of the lubricants to be used as well as the type of foreign materials to be repelled or achieving long-term stability of retained lubricant in a variety of host media and shear conditions including liquids, gas, and solid hosts by changing the nature of the ligands.

An illustrative ultra-repellant surface **200** is shown in **FIG. 1** (not drawn to scale). Referring to **FIG. 1**, an anchoring layer **210** of immobilized molecules that exhibit chemical properties that bind and retain an ultra-thin lubricating layer **220** composed of a liquid is attached to a substrate **230**. Substrate **230** has a smooth surface. The immobilized molecular anchoring layer is covalently attached to the substrate **230**. A lubricating liquid comprising a perfluorocarbon oil is applied to the surface-modified substrate. The surface modifying anchoring layer enhances the wetting properties of the lubricating liquid and allows it to form a thin lubricating layer. The immobilized molecular anchoring layer allows the lubricating liquid to be added to smooth or roughened substrate **230** and still repel immiscible materials. Repellent material **240** is the material to be repelled, and can be a liquid, particulate contained within a liquid, a complex fluid, a dry solid, or a solid surface. The selection of the lubricating liquid (and thus the composition of the underlying anchoring layer) is made to provide a lubricating layer in which the repellent material is immiscible.

The anchoring layer is covalently bound to the underlying surface, as is illustrated in **FIG. 2****.** A substrate contains an immobilized molecular anchoring layer that attaches to the surface. Referring to **FIG. 2**, the immobilized molecular anchoring layer **110** includes a head region **120** that provides a chemical linkage to the substrate **100.** The immobilized molecular anchoring layer **110** also includes a tail region **130.** The tail region of the immobilized molecular anchoring layer alters the surface properties of the substrate to provide a desired property. For example, depending on the nature of the repellent material, the immobilized molecular anchoring layer can increase the lipophobicity, hydrophobicity, or omniphobicity of the surface. The tail region interacts with e.g., solubilized molecules of the lubricating liquid that is applied to the treated surface. Thus, the tail region retains the molecules of the lubricating liquid by non-covalent attachment The tail region and molecules of the lubricating liquid are arranged on the surface such that the molecules of lubricating liquid form a lubricating layer **140** on the surface. Because of the affinity is based on the interaction of the lubricating liquid with the functional regions of the anchoring layer, the lubricating layer can be very thin and can be no more than one molecular layer.

The lubricating layer **140** is formed by immobilizing the anchoring layer **110** on the surface **100** and applying a lubricating liquid to the surface containing the immobilized monomolecular surface layer **110.** The lubricating liquid wets the treated surface of the substrate and forms the lubricating layer **140.** The anchoring layer **110** and lubricating layer **140** are held together by non-covalent attractive forces. Together, the substrate and lubricating layers on the substrate form a slippery surface that resists adhesion by molecules and particles, and repels certain immiscible fluids. This allows the passage of materials at various flow rates, including high flow rates, without allowing the material to adhere to, attach, foul the surface, or, in the case of biological fluids such as blood, coagulate. Thus, these surfaces can be used in a wide variety of environments, such as laboratories, on medical devices, medical equipment, for medical applications including anticoagulation and anti-biofilm formation, industrial applications, commercial applications, and other practical applications. As used herein, reference to an "environmental material" or "environmental liquid" indicates a fluid or solid or other material, for which the ultra slippery layer according to the disclosure is designed to repel or reduce adhesion. Other terms, such as "repellent material," "repellent liquid," "material to be repelled," "fluid to be repelled," "liquid to be repelled," and the like, are meant to denote such similar materials.

Perfluorocarbon ("PFC") oil is used as the lubricating liquid, particularly for the case when the materials to be repelled or excluded are immiscible in oleophobic liquids. The "Teflon-like" PFC oil is retained on the surface by a "Teflon-like" layer on the surface, *e.g*., a fluorous surface, which serves as the anchoring layer. The treated fluorous surface has an affinity for other fluorocarbons, and thus when PFC oil is applied to the treated surface, the surface is wetted by and retains a thin layer of PFC oil that resists adhesion of liquids and repels materials.

### Substrate

Many types of substrates can be used in accordance with this disclosure. Generally, solids having chemically reactive surfaces (or surfaces that can be activated to provide chemically reactive surfaces) can be used to interact with and immobilize the anchoring layer and the lubricating layer applied to the surface. In one embodiment, the surface is smooth. In other embodiments, the surface is not limited to any degree of surface roughness.

The liquid repellant surfaces disclosed herein have properties that are independent of the geometry of the underlying substrate. Thus, the geometry of the substrate can be any shape, form, or configuration to suit the configuration of a variety of materials. Non-limiting examples of shapes, forms, and configurations that liquid repellant surfaces can take include generally spherical (e.g., beads) (see **FIGS. 29** and **30**), tubular (e.g., for a cannula, connector, catheter, needle, capillary tube, or syringe) (see **FIG. 4A-4C**), planar (e.g., for application to a microscope slide, plate, wafer, film, or laboratory work surface) (see **FIGS. 5-26** and **FIG. 28**), or arbitrarily shaped (e.g., well, well plate, Petri dish, tile, jar, flask, beaker, vial, test tube, column, container, cuvette, bottle, drum, vat, or tank) (see **FIG. 32**). The substrate can be a solid that is flexible or rigid.

In some embodiments, the substrate is flexible, such as for example, a flexible tube or tubing used in medical applications. **FIGS. 4A-4C** show a flexible PDMS tubing that has been treated according to one or more embodiments of the invention and made liquid ultra-repellant.

The substrate can be any material that is capable of surface modification to form the immobilized molecular anchoring layer. Many suitable materials are commercially available, or can be made by a variety of manufacturing techniques known in the art. Non-limiting examples of surfaces that can be used to prepare the ultra-slippery surfaces described herein include, *e*.*g*., glass, polymers (e.g., polysulfone, polystyrene, polydimethylsiloxane ("PDMS"), polycarbonate, polymethylmethacrylate, polyethylene, polypropylene, polyethylene terephthalate, polyvinyl chloride, styrene-ethylene/butylene-styrene, styrene-ethylene/propylene-styrene, polyurethane, silicone, etc.), metals (e.g., stainless steel, nitinol, titanium, gold, platinum, silver, aluminum, cobalt-chrome, etc.), paper, plastics, various forms of carbon (e.g, diamond, graphite, black carbon, etc.), metal oxides and other ceramic materials, composite materials, combinations of the above, and the like.

In certain environments, the substrate is selected to be compatible with the intended use of the device. For example, in medical devices, it is preferred that the solid material comply with FDA standards for safety and biocompatibility.

Suitable substrates contain reactive surface moieties in their native forms, or can be treated to provide suitable reactive moieties for linking with the anchoring compound. Exemplary reactive surface moieties include oxygen-containing surface groups such as oxides, hydroxides, carboxyl, carbonyl, phenol, epoxy, quinone and lactone groups and the like; nitrogen-containing surface groups such as amino, C=N groups, azides, amides, nitrile groups, pyrrole-like structure and the like, sulfur-containing moieties such as thiols, and the like, and reactive carbon containing surface groups such as alkynes and alkenes.

Surfaces can be treated to activate the surface and render it amenable to surface modification using well-understood techniques. Exemplary surface treatments include acid or base treatment, oxidization, ammonization, plasma, microwave treatment, and various etching techniques.

### Anchoring Layer

According to the present disclosure, the substrate is modified by providing an anchoring layer that has an affinity for and an ability to retain a lubricating liquid, on the substrate. Materials known to have strong omniphobic properties do not adhere to or spread out well on most hydrophilic or hydrophobic substrates. Similarly, materials known to have strong hydrophobic properties do not adhere to or spread out well on most hydrophilic or omniphobic substrates, and materials known to have strong hydrophilic properties do not adhere to or spread out well on most hydrophobic or omniphobic substrates. The selection of the appropriate immobilized molecular anchoring layer can improve the wetting properties of such liquids and thereby provide a surface with excellent liquid repelling properties.

The anchoring layer comprises a head group that covalently attaches to the substrate, and a functional group that non-covalently interacts with the lubricating layer to retain the lubricating layer on the surface. This anchoring layer forms a monomolecular layer on the substrate. The anchoring layer can be prepared by reaction of a reactive head group ("R2" in **FIG. 3**) of a bifunctional molecule bearing the functional tail, with a reactive species ("R1" in **FIG. 3**) on the surface of the substrate **310.** The reaction of R2 and R1 forms a covalent linkage 320 that secures the functional group on the surface of the substrate. For example, reactive oxygen moieties on the surface ("R1") react with the trichlorosilane moieties ("R2") of a perfluorinated or polyfluorinated organosilane, to form a siloxy (Si-O) linkage and rendering a modified surface of exposed perfluorinated or polyfluorinated tails.

By way of example, the reactive head group (R2) is a group that reacts with oxygen-containing surface groups (R1) such as oxides, hydroxides, carboxyl, carbonyl, phenol, epoxy, quinone and lactone groups and the like; nitrogen-containing surface groups (R1) such as amino, C=N groups, amides, azides, nitrile groups, pyrrole-like structure and the like, sulfur-containing moieties such as thiols, and the like that are on the surface of the substrate, and reactive carbon containing surface groups such as alkynes and alkenes.

Some examples of groups that form upon reaction of R1 with R2 include ethers, silyl ethers, siloxanes, esters of carboxylic acids, esters of sulfonic acids, esters of sulfinic acids, esters of sulfuric acids, esters of phosphonic acids, esters of phosphinic acids, esters of phosphoric acids, silyl esters of carboxylic acids, silyl esters of sulfonic acids, silyl esters of sulfinic acids, silyl esters of sulfuric acids, silyl esters of phosphonic acids, silyl esters of phosphinic acids, silyl esters of phosphoric acids, oxides, sulfides, carbocycles, heterocycles with at least one oxygen atom, heterocycles with at least one nitrogen atom, heterocycles with at least one sulfur atom, heterocycles with at least one silicon atom, 'click' reactions-derived heterocycles, Diels-Alder reactions-derived carbocycles, Diels-Alder reactions-derived heterocycles, amides, imides, sulfides, thiolates, metal thiolates, urethanes, oximes, hydrazides, hydrazones, physisorbed or chemisorbed or otherwise non-covalently attached moieties, or combinations thereof.

Non-limiting examples for R2 include carboxylic acids, amines, halides, silanols, thiols, carbonyls, alcohols, phosphonic acids, sulfonic acids, inorganic oxides (e.g., silica, titania, alumina, zirconia, etc.), reactive metals (e.g., gold, platinum, silver), azides, alkenes and alkynes.

For example, the surfaces with hydroxyl groups (i.e., -OH) can be functionalized with various commercially available substances such as polyfluoroalkylsilanes (e.g., tridecafluoro-1,1,2,2-tetrahydrooctyl-trichlorosilane, heptadecafluoro-1,1,2,2-tetra-hydrodecyl trichlorosilane, etc.), alkylsilanes, aminosilanes (e.g., (3-aminopropyl)-triethoxysilane, 3-(2-aminoethyl)-aminopropyltrimethoxysilane), glycidoxysilanes (e.g., (3-glycidoxypropyl)-dimethyl-ethoxysilane), and (mercaptoalkyl)silanes (e.g., (3-mercaptopropyl)-trimethoxysilane). In certain embodiments, a variety of materials that have or can easily form oxides on the surface, such as silicon, glass, alumina, and organic polymers, can be activated to contain -OH functional groups using techniques such a plasma treatment. After activation, either vapor or solution deposition techniques can be used to attach various organosilyl moieties to the substrates. Organosilyl moieties can be chosen from perfluorinated, partially fluorinated or non fluorinated ones.

In certain embodiments, non-limiting examples for R2 include thiol groups that reacts with metal substrates, such as gold, copper, silver, platinum, palladium, rhodium, ruthenium, their alloys and intermetallic compounds.

In another embodiment, non-limiting list of exemplary reactive group (R2) includes substituted or unsubstituted carboxylic acids, substituted or unsubstituted sulfonic acids, substituted sulfinic acids, substituted sulfuric acids, substituted phosphonic acids, substituted phosphinic acids, substituted phosphoric acids, and their respective esters, or combinations thereof.

In one or more embodiments, the anchoring layer includes a perfluorocarbon tail having a tail length of at least one carbon. In specific embodiments, the perfluorocarbon tail can have a carbon length of 1-50, or 2-20 or 4-16 or 6-12. In one or more embodiments, the anchoring group head group is a siloxy group (Si-O) formed in the reaction of a reactive silane group, e.g., thrichlorosilane, with oxygen moieties on the substrate surface. A number of commercially available perfluorocarbon trichlorosilanes are available. As used herein, reference to a "silanized' surface indicates an anchoring layer in which the head group includes and Si-O linkage.

In other embodiments, crosslinking agents can be used to link the reactive surface with the anchoring layer molecules. For example, as shown below, bifunctional linkers such as epichlorohydrin, glutaraldehyde, adipic dihydrazide can attach hydroxyl-, amino- and carboxylic acid terminated compounds to their respectively activated surfaces.

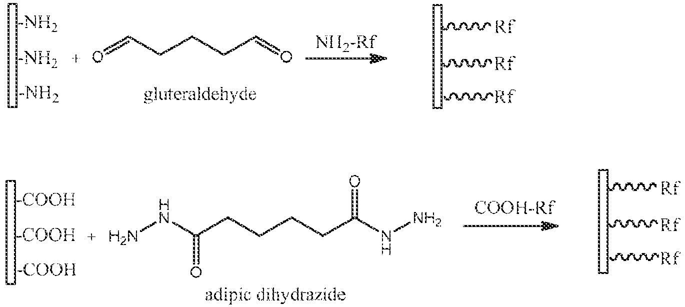

Table 1 shows additional examples of linking chemicals. A non-limiting list of exemplary linking reagents with the same or different reactive groups at either end are shown. The reagents are classified by which chemical groups link (left column) and their chemical composition (right column).

**Table 1**

| **Crosalinking Target** | **Linker Reactive Groups, Features** |
|---|---|
| Amine-to-Amine | NHS esters |
| | Imidoesters |
| Sulfhydryl-to-Sulfhydryl | Maleimides |
| Nonselective | Aryl azides |
| Amine-to-Sulfhydryl | NHS ester / Maleimide |
| | NHS ester / Pyridyldithiol |
| | NHS esters / Haloacetyl |
| Amine-to-Nonselective | NHS ester / Aryl Azide |
| | NHS ester / Diazirine |
| Amine-to-Carboxyl | Carbodiimide |
| Sulfhydryl-to-Carbohydrate | Maleimide / Hydrazide |
| | Pyridyldithiol / Hydrazide |
| Amine-to-DNA | NHS ester / Psoralen |

The functional group (tail) used in the anchoring layer can be selected to have an affinity, such as non-covalent interaction, with molecules of the lubricating layer and retain the lubricating layer on the surface. As used herein, high affinity refers to the spreading coefficient of the lubricant over that of the functional group is positive, such as having attractive forces and generally miscible with one another, such that the lubricating liquid has a greater adsorption equilibrium constant with the functional group of the anchoring layer than the material to be repelled does to the functional group of the anchoring layer. Particularly, a no-slip condition can develop between the lubricating liquid and the anchoring layer so that there is an outermost molecules of the lubricating liquid that is stuck to the anchoring layer although other parts of the lubricating liquid may be forced away from the substrate (e.g., shear deformation, high impact pressure, etc.) For example, functional groups comprising hydrocarbons such as alkanes, alkenes, alkynes, and aromatic compounds, and combinations thereof can be used to create a hydrophobic surface that has an affinity for lubricating liquids that are also hydrophobic or lypophilic. The combined surface layer and lubricating liquid is useful for repelling hydrophilic or omniphobic fluids. In another embodiment, hydrophilic functional groups can be used to create a hydrophilic surface that has an affinity for hydrophilic liquids. Exemplary hydrophilic groups include charged polypeptides, polyanions (e.g., heparin sulfate, oligonucleotides, dextran sulfate), polycations (e.g. chitosan, chitin, hexadimethrine bromide, diethylaminoethyl cellulose) polar polymers (polyacrylamide, polyethylene glycol, polypropylene glycol), polysaccharides (dextran, agarose, inulin, sepharose), amines (e.g. aminopropyl, diethylaminoethanol), carboxylic acids, guanidine, alcohols, sulfhydryls, carboxamides, and metal oxides. The combined surface layer and lubricating liquid is useful for repelling hydrophobic or omniphobic fluids. In still another embodiment, functional groups comprise perfluorinated groups (e.g., perfluoropoly (or oligo) ethers, etc.) that have affinity to lubricants to create an omniphobic surface for repelling hydrophilic or hydrophobic fluids.

The substrate can be coated with the anchoring layer by methods well known in the art, including plasma-assisted chemical vapor deposition, chemical functionalization, chemical solution deposition, chemical vapor deposition, chemical cross linking, and atomic layer deposition. For example, chemical vapor deposition can be carried out by exposing the substrate to reactive silane vapors. For chemical solution deposition, the deposition can be carried out by, e.g., immersing the substrate in a silane solution followed by rinsing and drying. Similarly, other reactive head groups can be brought in contact and made to react with the surface by using gas- and solution-phase methods well-established in the art.

The anchoring layer applied in a typical thickness is assumed to be a monomolecular layer, however, the layer may not completely cover the entire surface but still be sufficient to modify the surface properties of the substrate.

Certain embodiments may involve reacting perfluoroalkylamines, with carbon chain lengths ranging from ethyl to dodecyl, such as 1H,1H-perfluorooctylamine, to different surfaces like polyesters, polyurethanes, or polyvinylchloride through aminolysis of the esters and carbamates in the backbone or nucleophilic substitution.

In certain embodiments, the underlying substrate functionalized with the desired anchoring layer via a two-step process, such as by reacting the substrate surface to provide a desired reactive moiety, which can then be further reacted with the desired anchoring layer. For example, an underlying substrate (e.g., silica) can be reacted to provide an isocyanate group, which can then be utilized to carry out a carbamation reaction between hydroxyl or amino terminated fluoro compound (HO-Rf and NH₂-Rf) and isocyanatopropyl triethoxysilane (ICPTES) to form fluorosilane linker through urethane and urea formation respectively.

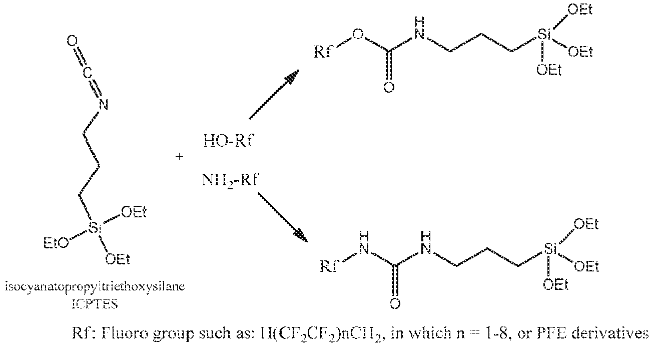

As another example, as shown below, hydroxylated surfaces can be functionalized with triethoxysilyl butyraldehyde (ABTES) and further reacted with amino terminated fluoro compounds.

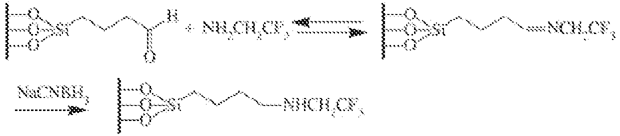

In certain embodiments, perfluoroalkylamines can react with surfaces bearing acrylates, maleimides, carboxylic acids, anhydrides, aldehydes, and epoxides through Michael addition, amidation, nucleophilic addition, and ring-opening mechanisms.

Other nucleophilic perfluorinated molecules include perfluoroalkylthiols such as perfluorodecanethiol that may react with electrophiles such as maleimides as well as disulfide-containing substrates.

In certain embodiments, perfluoroalkyl alcohols like 1H,1H,2H,2H-perfluoro-1-octanol could be anchored to carboxylic acid-containing substrates through esterification.

In certain embodiments, substrates containing amines and relevant nucleophiles can react with perfluoroalkylacrylates, ranging in carbon chain length from ethyl to dodecyl, such as 1,1,1,3,3,3-hexafluoroisopropyl acrylate, or perfluoroalkylepoxides, such as perfluorohexyl propyl epoxide or perfluorooctyl propyl epoxide.

In certain embodiments, perfluoroalkyliodides, with chain lengths ranging from ethyl to dodecyl, such as 2-(perfluorooctyl)ethyl iodide, may be reacted with olefin-bearing surfaces to yield iodide adducts in the presence of an amine and metal salt.

Hydrocarbon analogs of the aforementioned reactions may readily be obtained as well using fatty acids, lipids, alkylamines, alkanethiols, alkyl alcohols, alkyl halides, alkyl acrylates, and alkyl epoxides with varying carbon chain lengths, ranging from C2 to C22.

In certain embodiments, phosphonic acids can be utilized as part of the anchoring layer. As used herein, the term "phosphonic acid" refers to an organic compound having the structure: 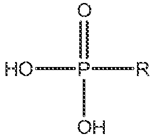 wherein R is an organic (carbon-containing) radical or residue wherein the phosphorus atom is bonded to a carbon atom of the R group. Those of ordinary skill in the art are aware that the hydrogens attached to the OH groups of phosphonic acids are acidic and can be removed by bases or at appropriate pH's to form salts of the phosphonic acids having phosphonate mono or di-anions having the structure:

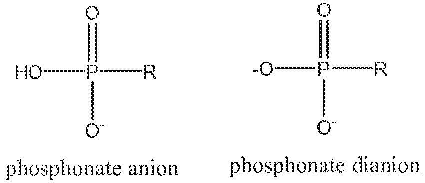

It is understood that, when present as an anion, the phosphate can include one or more associated counter ions, for example, monovalent cations including lithium, sodium, or potassium or one or more divalent cations including calcium or zinc. The organic "R" radical or residue comprises at least one carbon atom, and includes but is not limited to the many well-known carbon-containing groups, residues, or radicals well known to those of ordinary skill in the art. The R radicals can contain various heteroatoms, or be bonded to another molecule through a heteroatom, including oxygen, nitrogen, sulfur, phosphorus, or the like. Examples of suitable R radicals include but are not limited to alkyls such as methyl, butyl, or octadecyl radicals and the like, or substituted alkyls such as hydroxymethyls, haloalkyls, perfluoroalkyls, aromatics such as phenyls or substituted aromatics, such as phenols or anilines; or polymeric residues such as PEG, PPG, silicone, polyethylene, fluoropolymers such as perfluoropolyethers, Teflons or Vitons, polycarbonates, etc, and the like. In many non-polymeric embodiments, the R radicals of the phosphonates comprise 1 to 18 carbon atoms, 1 to 15, carbon atoms, 1 to 12 carbon atoms, 1 to 8 carbon atoms, or 1 to 4 carbon atoms.

In certain embodiments, phosphonic acid ligands can be attached to metal oxide substrate surfaces.

In another aspect, the phosphonic acid ligands can form a coating on the surface of metal oxide substrates.

In a further aspect, at least one phosphonic acid ligand comprises a residue of a compound having the structure Rₙ-Xₙ, wherein R is a ligand group and X is a phosphonic acid group having the structure: 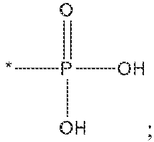 and
wherein each n is, independently, 1, 2, or 3.

In a yet further aspect, each n is 1. In a still further aspect, the compound comprises the structure R-X.

In one aspect, a chemically functionalized substrate of the invention can comprise at least one phosphonic acid ligand.

In a further aspect, a chemically functionalized substrate of the invention can comprise a plurality of phosphonic acid ligands.

In yet another aspect, a chemically functionalized substrate of the invention can be covered with phosphonic acid ligands.

In yet a further aspect, a chemically functionalized substrate of the invention can be covered with a mixture of more than one type of phosphonic acid ligands.

The term "phosphonic acid ligand" as used herein refers to a radical or residue attached to or capable of attaching to the surface of the metal oxide substrates that is derived from a phosphonic acid. Those of ordinary skill in the art will understand that phosphonic acids or their anionic salts can be readily attached to a surface of a metal oxide, by replacement of one or more of the oxygen atoms of the phosphonic acid with bonds ranging from covalent, to polar covalent, to ionic, and including through hydrogen bonding, between the phosphorus atom and an oxygen atom or ion on a metal oxide surface.

In certain aspects, at least one organic phosphonic acid comprises methylphosphonic acid, octylphosphonic acid, decylphosphonic acid, octadecylphosphonic acid, phenylphosphonic acid, benzylphosphonic acid, pentafluorobenzylphosphonic acid, 11-hydroxyundecylphosphonic acid, (11-phosphonoundecyl)phosphonic acid, (3,3,4,4,5,5,6,6,7,7,8,8,8-tridecafluorooctyl)phosphonic acid, pentabromobenzylphosphonic acid, (1 1-acryloyloxyundecyl)phosphonic acid, or a mixture thereof.

In one aspect, the phosphonic acid ligands are attached to the surface by bonding of one, two, or three of the oxygen atoms of the phosphonic acid ligands to the metal oxide surface. For example, the organic phosphonic acid ligands can be attached to the surface by bonds ranging from covalent, to polar covalent, to ionic, and including through hydrogen bonding, as illustrated by one or more of the structures illustrated below:

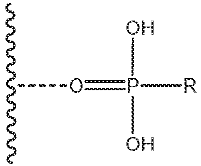

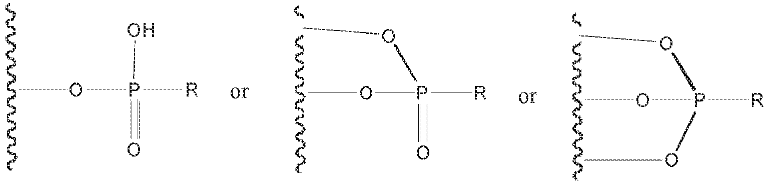

In one aspect, R can be an organic radical comprising 1 to 18 carbon atoms, for example, an organic radical comprising 1 to 16 carbons, 1 to 14 carbons, 1 to 12 carbons, 1 to 10 carbons, 1 to 8 carbons, 1 to 6 carbons, or 1 to 4 carbons.

In a further aspect, R is an alkyl substituted polyether having the structure: 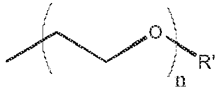 wherein n is 1 to 25 (including 1, 2, 3, 4, 5, 6, 7, 8, 9, 10, 11, 12, 13, 14, 15, 16, 17, 18, 19, 20, 21, 22, 23, 24, and 25), and R' is a C1-C4 alkyl (including 1, 2, 3, or 4 carbons). In a yet further aspect, R is selected from methyl, ethyl propyl, butyl, pentyl, hexyl, heptyl, octyl, nonyl, decyl, undecyl, and dodecyl.

In another aspect, R comprises a substituted or unsubstituted, linear or branched, C₃ to C₅₀ aliphatic or cyclic aliphatic, fluoroalkyl, oligo(ethyleneglycol), aryl, or amino group.

In another aspect, R can comprise linear or branched alkyl groups having up to 12 carbons (including 1, 2, 3, 4, 5, 6, 7, 8, 9, 10, 11, and 12 carbons) or having up to 8 carbons (including 1, 2, 3, 4, 5, 6, 7, and 8 carbons), and α and β can be, independently, integers from 1 to 12 (including 1, 2, 3, 4, 5, 6, 7, 8, 9, 10, 11, and 12) or integers from 1 to 8 (including 1, 2, 3, 4, 5, 6, 7, and 8).

In a further aspect, R is a fluorinated group. For example, R can comprise -(CH₂)_{β}-(OCH₂CH₂)_{α}F, -OCHCH₂-(CF₂)_{β}CF₃, -(CF₂CF₂)_{α}-(CF₂)_{β}CF₃, -(CF₂)_{β}-(CF₂CF₂)_{α}CF₃,-(CF₂CF₂)_{α}-(CH₂)_{β}CF₃, or -(CF₂)_{β}-(CF₂CF₂)_{α}CF₃, wherein α is an integer from 0 to 25 and wherein β is an integer from 0 to 25. In various further aspects, α and β can be, independently, integers from 1 to 12 (including 1, 2, 3, 4, 5, 6, 7, 8, 9, 10, 11, and 12) or integers from 1 to 8 (including 1, 2, 3, 4, 5, 6, 7, and 8).

### Lubricating Layer

The lubricating liquid used to form the lubricating layer is applied to the anchoring layer. Thus, the lubricating layer, which flows readily over the substrate, can stably, but non-covalently bind to the functional group of the anchoring layer to form a continuous, repellant layer. The lubricating layer is selected based on its ability to repel immiscible materials. In one or more embodiments, the lubricating layer is inert with respect to the underlying substrate and environmental material to be repelled.

The ultra-slippery surface is used in a medical setting, in which case the lubricating liquid is selected, e.g., based on its biocompatibility, level of toxicity, anticoagulation properties, and chemical stability under physiologic conditions. For example, compounds that are approved for use in biomedical applications can be used without being part of the present disclosure. Perfluorinated organic liquids, in particular, are suitable for use in biomedical applications. In the present disclosure, the lubricating layer is perflurocarbon PFC oils such as FC-43, FC-70, perfluorotripropylamine, perfluorotripentylamine, perfluorotributylamine, perfluorodecalin, perfluorooctane, perfluorobutane, perfluoropropane, perfluoropentane, perfluorohexane, perfluoroheptane, perfluorononane, perfluorodecane, perfluorododecane, perfluorooctyl bromide, perfluoro(2-butyl-tetrahydrofurane), perfluoroperhydrophenanthrene, perfluoroethylcyclohexane, perfluoro(butyltetrahydrofuran), perfluoropolyethers (KRYTOX), and combinations thereof. In other aspects, the lubricating layer is fluorinated hydrocarbon oil, non-limiting examples of which include oils such as 3-ethoxy-1,1,1,2,3,4,4,5,5,6,6,6-dodecafluoro-2-trifluoromethyl-hexane, trifluoromethane, difluoromethane, pentafluoroethane, hydrofluoroether, etc.

In some aspects, the viscosity of the lubricating layer can be chosen for particular applications. For example, the viscosity of the lubricating oil can be < 1 cSt, <10 cSt, < 100 cSt, < 1000 cSt, or <10,000 cSt

In some aspects, the lubricating layer has a low freezing temperature, such as less than -5 °C, -25 °C, or -50 °C. A lubricating layer with a low freezing temperature allows the layer to remain liquid in low temperatures to maintain the ability of the combination of the lubricating layer and functionalized surface to repel a variety of liquids or solidified fluids, such as ice and the like.

In some aspects, the lubricating layer has a low evaporation rate or a low vapor pressure. For example, the vapor pressure of the lubricating liquid can be less than 10 mmHg at 25 °C, less than 5 mmHg at 25 °C, less than 2 mmHg at 25 °C, less than 1 mmHg at 25 °C, less than 0.5 mmHg at 25 °C, or less than 0.1 mmHg at 25 °C. The lubricating layer can be applied in a thickness sufficient to cover the anchoring layer. In some embodiments, the lubricating layer is applied at a thickness sufficient to form a monomolecular layer on the substrate. In other embodiments, the lubricating layer is applied at a thickness of 10 nm to 10 µm on the substrate. In other embodiments, the lubricating layer is applied at a thickness of 10 µm to 10 mm on the substrate. The lubricating layer applied in a typical thickness, assumed to be a monomolecular layer, can remain liquid repellant for a long period without requiring replenishing. By way of example, the surface can remain liquid repellant for a period longer than 1 hour, or longer than 6 hours, or longer than 24 hours, longer than a week, or longer than a year or more.

The lubricating liquid can be sprayed, cast, or drawn onto the substrate either once or repeatedly. In certain embodiments, the lubricating layer can be applied to the surface by spinning coating, pipetting drops of lubricating liquid onto the surface, or dipping the surface into a reservoir or channel containing the lubricating liquid, through microscale holes in the wall of the underlying substrate, or by presaturating the surface with lubricating liquid to form a lubricating layer. The lubricating liquid can also be applied by absorption, wicking, thin layer deposition, or by intermittent passing of volumes of lubricating liquid over the surface (e.g., small plugs or bubbles flowing in a catheter). In some embodiments, any excess lubricating liquid can be removed by spinning the coated article or by drawing a squeegee across the surface or flushing and rising with another liquid.

In some embodiments, the lifetime of the liquid repellant surface can be extended by reapplying the lubricating layer at a certain interval. For example, **FIGS. 4A-4C** show a pump through which plugs of lubricating liquid **405** (shown as light colored areas in tubing) are periodically sent through PDMS tubing **410, 420** that was pre-treated with silane (see also, Example 2). In some aspects, the lubricating layer is replenished every 1, 5, 10, 15, 20, 30, 40, 50, or 60 seconds. In other aspects, the lubricating layer is replenished every 5, 10, 15, 20, 30, 40, 50, or 60 minutes. In still other aspects, the lubricating layer is replenished every 2, 4, 6, 8, 10, 12, 24, 48, 60, or 72 hours or more. Yet in other aspects, the lubricating liquid can be replenished continuously, at a constant or varying rate. In other embodiments, the surface can be replenished with lubricating liquid from a reservoir **160** housed below the substrate **100** as shown in **FIG. 2****.** The lubricating liquid is drawn through micropassages **150** to replenish lubricating liquid lost to the environment.

### Uses

In one or more embodiments, any arbitrary liquid (e.g., a biological fluid), and solid particulates contained therein, may be strongly repelled from the surfaces modified in accordance with the present disclosure. Similarly, adhesion of one solid surface to another solid surface can be prevented, or the friction between two solid surfaces can be reduced using the methods disclosed herein.

For example, **FIG. 31A** is a representation of an ultra-slippery surface **3100** including an anchoring layer **3110** and a lubricating layer **3120** on a substrate **3130** that is used to prevent an environmental liquid **3140**, and solutes and solid particulates **3145** contained in liquid **3140**, from adhering to underlying substrate **3130**. Thus, solutions and suspensions can be prevented from adhering to the surface of articles that have been coated with the ultra-slippery coating according to one or more embodiments. In other embodiments, the ultra-slippery surface **3100** provides a low friction interface with environmental liquid **3140** (and the entrained solutes and particles **3145**).

Medical disciplines ranging from cardiovascular medicine and oncology to orthopedics and ophthalmology rely increasingly on the implantation of medical devices into coronary arteries, jugular and femoral veins, joints, and many other parts of the body. Use of these devices risks the development of implant-induced thrombogenesis, or blood clotting. Similarly, blood processing equipment such as blood dialysis instruments, in particular, dialysis catheters, must take precautions to prevent blood clotting. In particular, blood naturally coagulates when exposed to glass. In one application, surfaces that normally contact blood can be coated with the ultra slippery coating described herein to reduce thrombogenesis, e.g., blood clotting and coagulation. As demonstrated in the examples below, ultra slippery coatings using a perfluorinated anchor layer and a perfluorohydrocarbon lubricating layer is highly effective in reducing thrombosis on surfaces that are in prolonged contact with unheparinized blood, even in flowing conditions.

In another embodiment, **FIG. 31B** shows an ultra-slippery surface **3155** used to prevent an environmental liquid **3160** from adhering to substrate **3130** surface, while simultaneously retaining selected solutes or particles on the surface. The ultra slippery surface includes an anchoring layer **3170** and a lubricating layer**3180**. In addition to immiscibility with respect to the environmental liquid **3160**, the lubricating layer **3180** is selected for its ability to dissolve or retain solutes or particulates **3165.** In one or more embodiments, the slippery surface serves as a selective filter that allows the solutes, particulates, or mixtures thereof contained in environmental liquid **3160** to adhere to, or be retained on, substrate, e.g., by dissolving or becoming suspended in lubricating layer **3180.** This selective affinity for components contained in environmental liquid **3160** can be achieved by, e.g., using a lubricating liquid in which components contained in the environmental liquid are miscible in Liquid C, or using a lubricating liquid that contains molecules that have an affinity for both the lubricating liquid and specific components contained in the environmental liquid, or molecules that are bound to the substrate having an affinity for specific components contained in the environmental liquid.

**FIG. 31C** illustrates the selective affinity of the ultra slippery surface **3155** for certain solutes and/or particulates. Substrate **3130** includes an anchoring layer **3170** that wets and binds lubricating layer **3180** containing a lubricating liquid. The slippery surface can be located, for example, on the inner surface of a tube through which an environmental liquid 3160 flows in the direction indicated by the arrow. The environmental liquid contains a first solute (solute 1) that has a low affinity for the lubricating liquid and a second solute (solute 2) that has a high affinity for the lubricating liquid. As the environmental liquid flows over the interface with the lubricating liquid, Solute 2 is preferentially adsorbed into the stationary liquid layer containing the lubricating liquid.

Selective affinity for components contained within a liquid to be repelled is useful in many situations. For example, selective affinity for components contained within a liquid can be useful for modifying chromatography columns to capture or bind desired molecules contained within a liquid passed through the column, but prevent the capture or binding of other molecules.

Blood naturally coagulates when exposed to glass. Therefore, it is particularly useful that glass slides and glass beads can be modified in accordance with the present disclosure to prevent blood clot formation and cell adhesion on surfaces while having selective affinity for certain blood components. When modified to allow selective affinity for blood components, such surfaces can be used to separate cells, pathogens, and other components from blood. Binding proteins, such as antibodies, lectins, and enzymes can be coupled to glass beads to attract desired free and bound blood components. For example, glass beads coupled to heparinase can be modified in accordance with the methods disclosed herein to attract and remove heparin while repelling blood and other components contained therein. Blood can also be passed through a chromatography column to remove biomolecules, such as autoantibodies, rheumatoid factors, and the like. Surfaces modified for selective affinity for blood components can be used in the dialysis context to remove components such as toxic metabolites before the blood is returned to the patient. Moreover, surfaces can be modified to detoxify blood of components, such as excess glucose present in the blood of diabetic patients.

Thus, the disclosed liquid repellant surfaces can be used in a number of biological applications, including preventing blood clotting, cell adhesion, and fouling of most surfaces. Moreover, these surfaces do not require anticoagulants when used to prevent blood clot formation.

In another embodiment, the surfaces described in accordance with the present disclosure can be used to prevent two substrates from adhering, or to reduce the friction between two substrates. **FIG. 27** is a schematic illustration of an ultra-slippery surface **2700** used to prevent a first substrate **2710** and a second substrate **2720** from sticking. Each of solid substrates, **2710, 2720,** possess an ultra slippery surface including anchoring layers **2730, 2735,** respectively, that interacts with and retains lubricating liquids, **2740, 2745**, respectively. Liquids **2740, 2745** are selected to be immiscible in one another. In addition, substrate **2710** has a preferential affinity for lubricating liquid **2740,**, while substrate **2720** has a preferential affinity for lubricating liquid **2745.** When substrates **2710, 2720** are in facing relationship with one another, the liquid/liquid interface defined at lubricating liquids, **2740, 2745** allows the friction between the substrates to be reduced.

**FIG. 32** (not belonging to the present disclosure) illustrates the application of the ultra slippery coating according to one or more embodiments to the interior surfaces of a food container (here, ketchup). The commercially available plastic bottle made of polyethylene terephthalate ("PETE") was silanized to form a fluorous surface and then treated with PFC oil. The coating prevented adhesion of the ketchup to the inner surface of the treated bottle (**FIG. 32B(ii)**) ad compared to an untreated bottle (**FIG. 32A(i)**).

In one or more of the above embodiments, non-limiting examples of surfaces that can be made liquid repellant include beads, cannula, connector, catheter (*e*.*g*., central line, peripherally inserted central catheter (PICC) line, urinary, vascular, peritoneal dialysis, and central venous catheters), catheter connector (*e.g*., Luer-Lok and needleless connectors), clamp, skin hook, cuff, retractor, shunt, needle, capillary tube, endotracheal tube, ventilator, associated ventilator tubing, drug delivery vehicle, syringe, microscope slide, plate, film, laboratory work surface, well, well plate, Petri dish, tile, jar, flask, beaker, vial, test tube, tubing connector, column, container, cuvette, bottle, drum, vat, tank, organ, organ implant, or organ component (*e.g.*, intrauterine device, defibrillator, corneal, breast, knee replacement, and hip replacement implants), artificial organ or a component thereof (e.g., heart valve, ventricular assist devices, total artificial hearts, cochlear implant, visual prosthetic, and components thereof), dental tool, dental implant (*e.g*., root form, plate form, and subperiosteal implants), biosensor (*e.g*., glucose and insulin monitor, blood oxygen sensor, hemoglobin sensor, biological microelectromechanical devices (bioMEMs), sepsis diagnostic sensor, and other protein and enzyme sensors), bioelectrode, endoscope (hysteroscope, cystoscope, amnioscope, laparoscope, gastroscope, mediastinoscope, bronchoscope, esophagoscope, rhinoscope, arthroscope, proctoscope, colonoscope, nephroscope, angioscope, thoracoscope, esophagoscope, laryngoscope, and encephaloscope), extracorporeal membrane oxygenation machines, heart-lung machines, surgical applications (*e.g*., sutures and vascular grafts), vascular applications (*e.g*., shunts), surgical patches (*e.g*., hernia patches), and combinations thereof.

In one embodiment, surfaces modified according to the present disclosure can repel a fluid without causing surface adhesion, surface-mediated clot formation, coagulation or aggregation. Non-limiting examples of biological fluids include water, whole blood, plasma, serum, sweat, feces, urine, saliva, tears, vaginal fluid, prostatic fluid, gingival fluid, amniotic fluid, intraocular fluid, cerebrospinal fluid, seminal fluid, sputum, ascites fluid, pus, nasopharengal fluid, wound exudate fluid, aqueous humour, vitreous humour, bile, cerumen, endolymph, perilymph, gastric juice, mucus, peritoneal fluid, pleural fluid, sebum, vomit, synthetic fluid (e.g., synthetic blood, hormones, nutrients), and combinations thereof.

In another embodiment, surfaces modified according to the present disclosure can repel various types of bacteria. In one embodiment, the type of bacteria repelled by these surfaces is gram positive bacteria. In another embodiment, the type of bacteria repelled by the disclosed modified surfaces is a gram negative bacterium. Non-limiting examples of bacteria repelled by surfaces modified in accordance with the present disclosure include members of the genus selected from the group consisting of *Actinobacillus* (*e.g*., *Actinobacillus actinomycetemcomitans*), *Acinetobacter* (*e.g*., *Acinetobacter baumannii*), *Aeromonas, Bordetella* (*e.g*., *Bordetella pertussis*, *Bordetella bronchiseptica*, and *Bordetella parapertussis*), *Brevibacillus, Brucella, Bacteroides* (*e.g*., *Bacteroides fragilis*), *Burkholderia* (*e.g*., *Burkholderia cepacia* and *Burkholderia pseudomallei*), *Borelia* (*e.g*., *Borelia burgdorfen*), *Bacillus* (*e.g*., *Bacillus anthracis* and *Bacillus subtilis*), *Campylobacter* (*e*.*g*., *Campylobacter jejuni*), *Capnocytophaga*, *Cardiobacterium* (*e.g*., *Cardiobacterium hominis*), *Citrobacter, Clostridium* (*e.g*., *Clostridium tetani* or *Clostridium difficile*), *Chlamydia* (*e.g*., *Chlamydia trachomatis*, *Chlamydia pneumoniae*, and *Chlamydia psiffaci*), *Eikenella* (*e.g*., *Eikenella corrodens*), *Enterobacter*, *Escherichia* (*e.g*., *Escherichia coli*), *Francisella* (*e.g*., *Francisella tularensis*), *Fusobacterium*, *Flavobacterium*, *Haemophilus* (*e.g*., *Haemophilus ducreyi* or *Haemophilus influenzae*), *Helicobacter* (*e.g*., *Helicobacter pylori*), *Kingella* (*e.g*., *Kingella kingae*), *Klebsiella* (*e.g*., *Klebsiella pneumoniae*), *Legionella* (*e.g*., *Legionella pneumophila*), *Listeria* (*e.g*., *Listeria monocytogenes*), *Leptospirae*, *Moraxella* (*e.g*., *Moraxella catarrhalis*), *Morganella*, *Mycoplasma* (*e.g*., *Mycoplasma hominis* and *Mycoplasma pneumoniae*), *Mycobacterium* (*e.g*., *Mycobacterium tuberculosis* or *Mycobacterium leprae*), *Neisseria* (*e.g*., *Neisseria gonorrhoeae* or *Neisseria meningitidis*), *Pasteurella* (*e.g*., *Pasteurella multocida*), *Proteus* (*e.g*., *Proteus vulgaris* and *Proteus mirablis*), *Prevotella*, *Plesiomonas* (*e.g*., *Plesiomonas shigelloides*), *Pseudomonas* (*e.g*., *Pseudomonas aeruginosa*), *Providencia*, *Rickettsia* (*e.g*., *Rickettsia rickettsii* and *Rickettsia typhi*), *Stenotrophomonas* (*e*.*g*., *Stenotrophomonas maltophila*), *Staphylococcus* (*e.g*., *Staphylococcus aureus* and *Staphylococcus epidermidis*), *Streptococcus* (*e*.*g*., *Streptococcus viridans*, *Streptococcus pyogenes* (group A), *Streptococcus agalactiae* (group B), *Streptococcus bovis*, and *Streptococcus pneumoniae*), *Streptomyces* (*e.g*., *Streptomyces hygroscopicus*), *Salmonella* (*e.g*., *Salmonella enteriditis*, *Salmonella typhi*, and *Salmonella typhimurium*), *Serratia* (*e.g*., *Serratia marcescens*), *Shigella*, *Spirillum* (*e*.*g*., *Spirillum minus*), *Treponema* (*e.g*., *Treponema pallidum*), *Veillonella*, *Vibrio* (*e.g*., *Vibrio cholerae*, *Vibrioparahaemolyticus*, and *Vibrio vulnificus*), *Yersinia* (*e.g*., *Yersinia enterocolitica*, *Yersinia pestis*, and *Yersinia pseudotuberculosis*), *Xanthomonas* (*e.g*., *Xanthomonas maltophilia*) and combinations thereof.

Surfaces modified according to the present disclosure can repel various types of fungi. Non-limiting examples of fungi repelled by modified surfaces include members of the genus *Aspergillus* (*e.g*., *Aspergillus flavus*, *Aspergillus fumigatus*, *Aspergillus glaucus*, *Aspergillus nidulans*, *Aspergillus niger*, and *Aspergillus terreus*), *Blastomyces dermatitidis*, *Candida* (*e.g*., *Candida albicans*, *Candida glabrata*, *Candida tropicalis*, *Candida parapsilosis*, *Candida krusei,* and *Candida guillermondii*), *Coccidioides immitis*, *Cryptococcus* (*e.g*., *Cryptococcus neoformans*, *Cryptococcus albidus*, and *Cryptococcus laurentii*), *Histoplasma capsulatum* var. *capsulatum*, *Histoplasma capsulatum* var. *duboisii*, *Paracoccidioides brasiliensis*, *Sporothrix schenckii*, *Absidia corymbifera*; *Rhizomucor pusillus*, *Rhizopus arrhizous*, and combinations thereof.

Surfaces modified according to the present disclosure can also repel various types of viruses and virus-like particles. In one or more embodiments, the virus repelled by these surfaces is selected from the group consisting of dsDNA viruses, ssDNA viruses, dsRNA viruses, (+)ssRNA viruses, (-)ssRNA viruses, ssRNA-RT viruses, dsDNA-RT viruses, and combinations thereof. Non-limiting examples of viruses repelled by surfaces modified in accordance with the present disclosure include cytomegalovirus (CMV), dengue, Epstein-Barr, Hantavirus, human T-cell lymphotropic virus (HTLV I/II), Parvovirus, hepatitides (e.g., hepatitis A, hepatitis B, and hepatitis C), human papillomavirus (HPV), human immunodeficiency virus (HIV), acquired immunodeficiency syndrome (AIDS), respiratory syncytial virus (RSV), Varicella zoster, West Nile, herpes, polio, smallpox, yellow fever, rhinovirus, coronavirus, Orthomyxoviridae (influenza viruses) (*e.g*., Influenzavirus A, Influenzavirus B, Influenzavirus C, Isavirus and Thogotovirus), and combinations thereof.

In still another embodiment, surfaces modified according to the present disclosure are capable of repelling particles in suspension or solution without causing surface adhesion, surface-mediated clot formation, coagulation, fouling, or aggregation. The omniphobic nature of the disclosed modified surfaces allows them to protect materials from a wide range of contaminants. Non-limiting examples of particles in suspension or solution include cells (e.g., normal cells, diseased cells, parasitized cells, cancer cells, foreign cells, stem cells, and infected cells), microorganisms (e.g., viruses, virus-like particles, bacteria, bacteriophages), proteins and cellular components (e.g., cell organelles, cell fragments, cell membranes, cell membrane fragments, viruses, virus-like particles, bacteriophage, cytosolic proteins, secreted proteins, signaling molecules, embedded proteins, nucleic acid/protein complexes, nucleic acid precipitants, chromosomes, nuclei, mitochondria, chloroplasts, flagella, biominerals, protein complexes, and minicells).

In yet another embodiment, commercially available devices (e.g., medical-grade apparatus or components) can be treated according to certain aspects of the present disclosure. For example, medical-grade PVC tubes can be treated so that their inner surfaces can possess certain repellant characteristics described in the present disclosure. In one or more embodiments, the surfaces are treated to reduce clotting in blood flowing through the medical tubing.

In some situations, the surfaces can be sterilized before or after the treatment. The ultra slippery coatings as described herein have been demonstrated to be sufficiently robust that they can maintain their slip charateristics, even after sterilization. The surface treatment (e.g., silanization) can be stable or robust enough that the surface maintains its repellant characteristics after an extended period of time (e.g., a day, week, a month, or more) and/or with sterilization process.

### EXAMPLES

The following examples are presented for the purpose of illustration only and are not intended to be limiting.

### Example 1

A silanized PDMS treated with PFC oil (Sigma Fluorinert® FC-70, Product Number F9880) was found to prevent adhesion and coagulation of blood without anticoagulant.

Perfluorocarbon silane (tridecafluorotetrahydrooctyltrichlorosilane, Sigma) was vapor deposited onto a PDMS sheet (Sylgard 184®, Dow Corning) and PDMS tubing (16 in length, 1.52 mm inner diameter, peroxide-cured silicone, Cole Parmer) over 10 hours under vacuum. Silanized and unsilanized PDMS sheets were coated with PFC oil by application of PFC from a pipette (perfluorotripentylamine, Sigma) (see **FIGS. 5B(ii)** and **5A(ii)**, respectively).

A 75 microliter volume of human blood free of anticoagulant was pipetted onto the PDMS sheets and the sheet was tilted. As shown in **FIG. 5**, blood adhered to the untreated and unmodified PDMS surface (**FIG. 5A(i)**), PDMS with perfluorotripentylamine (PFC oil) (**FIG. 5A(ii)**), and silanized PDMS (**FIG. 5B(i)**). However, blood did not adhere to, and was successfully repelled from, silanized PDMS with perfluorotripentylamine (PFC oil) (**FIG. 5B(ii)**). Silanized PDMS with PFC oil successfully repelled blood for over 90 min without replenishing the PFC oil. Blood repellency was also shown with perfluorodecalin as the PFC oil on a silanized PDMS sheet (not shown).

### Example 2

Silanized PDMS tubing treated as describe din Example 1 was shown to successfully prevent adhesion and coagulation during peristaltic pumping of blood through the tubing. Referring to **FIG. 4A**, the insides of both silanized **410** and unsilanized **420** PDMS tubing were coated with 0.5 mL PFC oil. Blood was pumped through the tubing at a rate of about 100 microliters/min for 45 minutes. A plug of PFC oil was pumped through the silanized tubing. No blood was visible in the plug of PFC oil, demonstrating that the blood had not yet bound to the surface of the tubing. Then 0.5 mL of PFC oil and 0.5 mL of deionized water were pumped through both sets of tubing.

Referring to **FIG. 4B**, after 3 min, blood had not coated the inside of the silanized tubing **410** through which blood and plugs of PFC oil were pumped, as demonstrated by the clear droplets of PFC oil that remained visible. Comparatively, significantly more binding of blood components was observed in the unsilanized tubing **420** compared to the silanized **410** tubing.

After 45 min of blood flow, PFC oil followed by water was pumped through both sets of tubing. As shown by **FIG. 4C**, silanized PFC oil-coated PDMS tubing **410** showed significantly less binding of blood than unsilanized PFC oil-coated PDMS tubing 420 even after being flushed with water and blood.

### Example 3

The ability of functionalized PDMS treated with PFC oil to repel liquid was investigated and compared to the liquid repellency of untreated and unmodified PDMS, PDMS treated with PFC oil, and silanized PDMS. Four sheets of PDMS (Dow Corning Sylgard® 184) were cured at 60 °C on mirror-polished aluminum. Two sheets of PDMS were silanized overnight by vacuum vapor deposition for approximately 12 hours using trichloro(1H,1H,2H,2H-perfluorooctyl)silane (Sigma, Product Number 448931). Once silanized, 250 µL PFC oil was applied to one of the two silanized PDMS sheets and to one unmodified PDMS sheet. Seventy-five µL of human blood without anticoagulant was applied to all four surfaces (see **FIG. 6A**).

The surfaces were then imaged. Once imaged, the PDMS sheets were tilted by hand and immediately reimaged as shown in **FIG. 6B. FIG. 6** shows that blood adhered to untreated and unmodified PDMS (**FIG. 6B(i)**), PDMS with PFC oil (**FIG. 6B(ii)**), and silanized PDMS (**FIG. 6B(iii)**), but was completely repelled by silanized PDMS with PFC oil (**FIG. 6B****(iv)**).

### Example 4

The ability of functionalized acrylic treated with PFC oil to repel liquid was investigated by comparing this ability to that of untreated and unmodified acrylic, acrylic treated with PFC oil, and silanized acrylic. Four sheets of Clear Cast Acrylic Sheet, 0.060" Thick, (McMaster Carr, Product Number 8560K171) were further modified with an oxygen plasma treatment at 500 mTorr for 40 seconds. Two sheets of acrylic were silanized overnight with trichloro(1H,1H,2H,2H-perfluorooctyl)silane under vacuum for approximately 13 hours.

Two-hundred and fifty µL of FC-70 was applied to one silanized and one unsilanized sheet of acrylic to create a "PFC-oiled" surface. Seventy-five µL of human blood without anticoagulant was applied to all four surfaces (see **FIG. 7A**).

The surfaces were imaged, then tilted by hand and immediately imaged again as shown in **FIG. 7B. FIG. 7** shows that blood adhered to untreated and unmodified acrylic (**FIG. 7B(i)**), acrylic with PFC oil (**FIG. 7B(ii)**), and silanized acrylic (**FIG. 7B(iii)**), but was completely repelled by silanized acrylic with PFC oil (**FIG. 7B****(iv)**).

### Example 5

The ability of functionalized tissue-culture polystyrene to repel liquid was investigated by comparing this ability to that of untreated and unmodified tissue-culture polystyrene, tissue-culture polystyrene treated with PFC oil, and silanized tissue-culture polystyrene. Four sheets of tissue-culture polystyrene previously treated with plasma by manufacturer (BD Biosciences, Product Number 353025) were used in this experiment. Two of the four sheets of tissue-culture polystyrene were silanized overnight with trichloro(1H,1H,2H,2H-perfluorooctyl)silane under vacuum for approximately 13 hours.

Two-hundred and fifty µL of FC-70 was applied to one silanized and one unsilanized sheet of tissue-culture polystyrene to create a "PFC-oiled" surface. Seventy-five µL of human blood without anticoagulant was applied to all four surfaces (see **FIG. 8A**).

The surfaces were imaged, then tilted by hand and immediately imaged again as shown in **FIG. 8B. FIG. 8** shows that blood adhered to untreated and unmodified tissue-culture polystyrene (**FIG. 8B(i)**), tissue-culture polystyrene with PFC oil (**FIG. 8B(ii)**), and silanized tissue-culture polystyrene (**FIG. 8B(iii)**), but was completely repelled by silanized tissue-culture polystyrene with PFC oil (**FIG. 8B****(iv)**).

### Example 6

The ability of functionalized polystyrene to repel liquid was investigated by comparing this ability to that of untreated and unmodified polystyrene, polystyrene treated with PFC oil, and silanized polystyrene. Four sheets of 1/32" thick polystyrene (McMaster Carr, Product Number 8734K29) were used in this experiment. The sheets were further modified with an oxygen plasma treatment at 500 mTorr for 40 seconds. Two sheets of the four sheets of polystyrene were silanized overnight with trichloro(1H,1H,2H,2H-perfluorooctyl)silane under vacuum for approximately 13 hours.

Two-hundred and fifty µL of FC-70 was applied to one silanized and one unsilanized sheet of polystyrene to create a "PFC-oiled" surface. Seventy-five µL of human blood without anticoagulant was applied to all four surfaces (see **FIG. 9A**).

The surfaces were imaged, then tilted by hand and immediately imaged again as shown in **FIG. 9B. FIG. 9** shows that blood adhered to untreated and unmodified polystyrene

(**FIG. 9B(i)**), polystyrene with PFC oil (**FIG. 9B(ii)**), and silanized polystyrene (**FIG. 9B(iii)**), but was completely repelled by silanized polystyrene with PFC oil (**FIG. 9B****(iv)**).

### Example 7

The ability of functionalized titanium treated with PFC oil to repel liquid was investigated and compared to that of untreated and unmodified titanium, titanium treated with PFC oil, and silanized titanium. Four sheets of titanium were further modified with an oxygen plasma treatment at 500 mTorr for 40 seconds. Two sheets of titanium were silanized overnight with trichloro(1H,1H,2H,2H-perfluorooctyl)silane under vacuum for approximately 13 hours.

Two-hundred and fifty µL of FC-70 was applied to one silanized and one unsilanized sheet of titanium to create a "PFC-oiled" surface. Seventy-five µL of human blood without anticoagulant was applied to all four surfaces (see **FIG. 10A**).

The surfaces were imaged, then tilted by hand and immediately imaged again as shown in **FIG. 10B. FIG. 10** shows that blood adhered to untreated and unmodified titanium (**FIG. 10B(i)**), titanium with PFC oil (**FIG. 10B(ii)**), and silanized titanium (**FIG. 10B(iii)**), but was completely repelled by silanized titanium with PFC oil (**FIG. 10B****(iv)**).

### Example 8

The ability of soda lime glass slides treated with PFC oil to repel liquid was investigated by comparing this ability to that of untreated and unmodified soda lime glass slides, soda lime glass treated with PFC oil, and silanized soda lime glass. Four soda lime glass slides (Corning, Product Number 2947-75x50) were further modified with an oxygen plasma treatment at 500 mTorr for 40 seconds. Two soda lime glass slides were silanized overnight with trichloro(1H,1H,2H,2H-perfluorooctyl)silane under vacuum for approximately 13 hours.

Two-hundred and fifty µL of FC-70 was applied to one silanized and one unsilanized soda lime glass slide to create a "PFC-oiled" surface. Seventy-five µL of human blood without anticoagulant was applied to all four surfaces (see **FIG. 11A**).

The surfaces were imaged, then tilted by hand and immediately imaged again as shown in **FIG. 11B. FIG. 11** shows that blood adhered to untreated and unmodified soda lime glass (**FIG. 11B(i)**), soda lime glass with PFC oil (**FIG. 11B(ii)**), and silanized soda lime glass (**FIG. 11B(iii)**), but was completely repelled by silanized soda lime glass with PFC oil (**FIG.**

### 11B(iv)).

### Example 9

The ability of functionalized polypropylene treated with PFC oil to repel liquid was investigated and compared to that of untreated and unmodified polypropylene, polypropylene treated with PFC oil, and silanized polypropylene. Four rectangular, 1/8" thick polypropylene bars (McMaster Carr, Product Number 8782K31) were further modified with an oxygen plasma treatment at 500 mTorr for 60 seconds. Two bars of polypropylene were silanized overnight with trichloro(1H,1H,2H,2H-perfluorooctyl)silane for approximately 13 hours.

Two-hundred and fifty µL of FC-70 was applied to one silanized and one unsilanized polypropylene bar to create a "PFC-oiled" surface. Seventy-five µL of human blood without anticoagulant was applied to all four surfaces (see **FIG. 12A**).

The surfaces were imaged, then tilted by hand and immediately imaged again as shown in **FIG. 12B. FIG. 12** shows that blood adhered to untreated and unmodified polypropylene (**FIG. 12B(i)**), polypropylene with PFC oil (**FIG. 12B(ii)**), and silanized polypropylene (**FIG. 12B(iii)**), but was completely repelled by silanized polypropylene with PFC oil (**FIG. 12B****(iv)**).

### Example 10

The ability of functionalized tape treated with PFC oil to repel liquid was investigated by comparing this ability to that of untreated and unmodified tape, tape treated with PFC oil, and silanized tape. Four sheets of polypropylene with acrylic adhesive (McMaster Carr, Product Number 75495A36) were silanized adhesive side up overnight with trichloro(1H,1H,2H,2H-perfluorooctyl)silane under vacuum for approximately 13 hours.

Two-hundred and fifty µL of FC-70 was applied to the adhesive side of one silanized and one unsilanized sheet of tape to create a "PFC-oiled" surface. Seventy-five µL of human blood without anticoagulant was applied to the adhesive side of all four surfaces (see **FIG. 13A**).

The surfaces were imaged, then tilted by hand and immediately imaged again as shown in **FIG. 13B. FIG. 13** shows that blood adhered to untreated and unmodified tape (**FIG. 13B(i)**), tape with PFC oil (**FIG. 13B(ii)**), and silanized tape (**FIG. 13B(iii)**), but was completely repelled by silanized tape with PFC oil (**FIG. 13B****(iv)**).

### Example 11

The ability of functionalized silicon wafer treated with PFC oil to repel liquid was investigated and compared to that of untreated and unmodified silicon wafer, silicon wafer treated with PFC oil, and silanized silicon wafer. The polished side of two silicon prime wafers (University Wafer) was further modified with an oxygen plasma treatment at 500 mTorr for 40 seconds. One silicon wafer was silanized overnight with trichloro(1H, 1H,2H,2H-perfluorooctyl)silane under vacuum for approximately 13 hours.

Two-hundred and fifty µL of FC-70 was applied to approximately one half of the silanized wafer and one half of the unsilanized wafer to create "PFC-oiled" surfaces. Seventy-five µL of human blood without anticoagulant was applied to all both halves of the two surfaces (see **FIG. 14A**).

The surfaces were imaged, then tilted by hand and immediately imaged again as shown in **FIG. 14B. FIG. 14** shows that blood adhered to untreated and unmodified half of silicon wafer (**FIG. 14B(i)**), the silicon wafer half treated with PFC oil (**FIG. 14B(ii)**), and silanized half of silicon wafer (**FIG. 14B(iii)**), but was completely repelled by silanized half of silicon wafer with PFC oil (**FIG. 14B****(iv)**).

### Example 12

The ability of functionalized polycarbonate treated with PFC oil to repel liquid was investigated by comparing this ability to that of untreated and unmodified polycarbonate, polycarbonate treated with PFC oil, and silanized polycarbonate. Four sheets of 1/8" thick scratch-resistant clear polycarbonate (McMaster Carr, Product Number 8707K111) were further modified with an oxygen plasma treatment at 500 mTorr for 40 seconds. Two sheets of polycarbonate were silanized overnight with trichloro(1H,1H,2H,2H-perfluorooctyl)silane under vacuum for approximately 13 hours.

Two-hundred and fifty µL of FC-70 was applied to one silanized and one unsilanized sheet of polycarbonate to create a "PFC-oiled" surface. Seventy-five µL of human blood without anticoagulant was applied to all four surfaces (see **FIG. 15A**).

The surfaces were imaged, then tilted by hand and immediately imaged again as shown in **FIG. 15B. FIG. 15** shows that blood adhered to untreated and unmodified polycarbonate (**FIG. 15B(i)**), polycarbonate with PFC oil (**FIG. 15B(ii)**), and silanized polycarbonate (**FIG. 15B(iii)**), but was completely repelled by silanized polycarbonate with PFC oil (**FIG. 15B****(iv)**).

### Example 13

The ability of functionalized polysulfone treated with PFC oil to repel liquid was investigated and compared to that of untreated and unmodified polysulfone, polysulfone treated with PFC oil, and silanized polysulfone. Four sheets of polysulfone (McMaster Carr) were further modified with an oxygen plasma treatment at 500 mTorr for 40 seconds. Two sheets of polysulfone were silanized overnight with trichloro(1H,1H,2H,2H-perfluorooctyl)silane under vacuum for approximately 13 hours.

Two-hundred and fifty µL of FC-70 was applied to one silanized and one unsilanized sheet of polysulfone to create a "PFC-oiled" surface. Seventy-five µL of human blood without anticoagulant was applied to all four surfaces (see **FIG. 16A**).

The surfaces were imaged, then tilted by hand, and immediately imaged again as shown in **FIG. 16B. FIG. 16** shows that blood adhered to untreated and unmodified polysulfone (**FIG. 16B(i)**), polysulfone with PFC oil (**FIG. 16B(ii)**), and silanized polysulfone (**FIG. 16B(iii)**), but was completely repelled by silanized polysulfone with PFC oil (**FIG. 16B****(iv)**).

### Example 14

The effect of PDMS surface roughness on creating a slippery surface was investigated. Four sheets of smooth PDMS were cured at 60 °C on Super Corrosion Resistant Stainless Steel (Type 316), #8 Mirror Finish (McMaster Carr, Product Number 9759K11) with an average roughness of 0.1 micrometers. Two PDMS sheets were then silanized (trichloro(1H,1H,2H,2H-perfluorooctyl)silane, Sigma, Product Number 448931)) overnight for approximately 12 hours. One sheet of silanized smooth PDMS and one sheet of smooth unsilanized PDMS were coated with 250 µL PFC oil (Sigma Fluorinert® FC-70, Product Number F9880) by (see **FIGS. 17A(ii)** and **17A(iv)**, respectively).

A 75 microliter volume of human blood free of anticoagulant was pipetting onto all four PDMS sheets. As shown in **FIG. 17**, The surfaces were imaged, then tilted by hand and immediately imaged again as shown in **FIG. 17B. FIG. 17** shows that blood adhered to untreated and unmodified smooth PDMS (**FIG. 17B(i)**), smooth PDMS with PFC oil (**FIG. 17B(ii)**), and silanized smooth PDMS (**FIG. 17B(iii)**), but was completely repelled by silanized smooth PDMS with PFC oil (**FIG. 17B****(iv)**).

Four sheets of rough PDMS similarly prepared as shown in **FIG. 18****.** Four sheets of rough PDMS were cured at 60 °C on Super Corrosion Resistant Stainless Steel (Type 316), #4 Satin Finish (McMaster Carr, Product Number 9745K11) with an average roughness of 1.0 micrometers. Two PDMS sheets were silanized (trichloro(1H,1H,2H,2H-perfluorooctyl)silane, Sigma, Product Number 448931)) overnight for approximately 12 hours. One sheet of silanized rough PDMS and one sheet of unsilanized rough PDMS were coated with 250 µL PFC oil (Sigma Fluorinert® FC-70, Product Number F9880) (see **FIGS. 18A(ii)** and **18A(iv)**, respectively).

A 75 microliter volume of human blood free of anticoagulant was pipetting onto all four PDMS sheets. As shown in **FIG. 18**, the surfaces were imaged, then tilted by hand and immediately imaged again as shown in **FIG. 18B. FIG. 18** shows that blood adhered to untreated and unmodified rough PDMS (**FIG. 18B(i)**), rough PDMS with PFC oil (**FIG. 18B(ii)**), and silanized rough PDMS (**FIG. 18B(iii)**), but was completely repelled by silanized rough PDMS with PFC oil (**FIG. 18B****(iv)**).

A rougher grade of PDMS cured at 60 °C on Super Corrosion Resistant Stainless Steel (Type 316), #2B Mill Finish (McMaster Carr, Product Number 88885K12) with an average roughness of 2.0 micrometers was similarly tested as shown in **FIG. 19****.** The sheets were silanized and 75 microliters of anticoagulant-free blood was pipetted onto the sheets. The surfaces were imaged, then tilted by hand and immediately imaged again as shown in **FIG. 19B. FIG. 19** shows that blood adhered to untreated and unmodified rougher PDMS (**FIG. 19B(i)**), rougher PDMS with PFC oil (**FIG. 19B(ii)**), and silanized rougher PDMS (**FIG. 19B(iii)**), but was completely repelled by silanized rougher PDMS with PFC oil (**FIG. 19B****(iv)**).

A comparison of **FIGS. 17B****(iv)** (smooth PDMS), **18B(iv)** (rough PDMS), and **19B(iv)** (rougher PDMS) shows that anticoagulant-free human blood was completely repelled by silanized PDMS with PFC oil without regard to the smoothness of the PDMS material used.

### Example 15

Silanes with tails of different fluorocarbon chain lengths were used to determine whether fluorocarbon chain length affects the ability of a surface to repel liquids and materials. Referring to **FIG. 20**, two slides of soda lime glass were modified with an oxygen plasma treatment for 40 seconds. One glass slide was coated with 250 µL PFC oil (Sigma Fluorinert® FC-70, Product Number F9880), and 75 microliters of anticoagulant-free blood was pipetted onto both slides. The surfaces were imaged, then tilted by hand and immediately imaged again as shown in **FIG. 20B****.** Blood adhered to both the plasma-treated glass side without PFC oil (**FIG. 20B(i)**) and the plasma-treated glass slide with PFC oil (**FIG. 20B(ii)**).

This process was repeated with plasma-treated, 1-PFC-treated soda lime glass slides. Referring to **FIG. 21**, two slides of soda lime glass were modified with an oxygen plasma treatment for 40 seconds and silanized (∼2 hours) (Trifluoropropyltrichlorosilane, Gelest, SIT8371.0) to achieve 1-fluoridated carbon-treated glass. One glass slide was coated with 250 µL PFC oil (Sigma Fluorinert® FC-70, Product Number F9880), and 75 microliters of anticoagulant-free blood was pipetted onto both slides. The surfaces were imaged, then tilted by hand and immediately imaged again as shown in **FIG. 21B****.** The blood adhered to the plasma-treated 1-PFC-treated glass side without PFC oil (**FIG. 21B(i)**). Contrastingly, much of the blood was repelled on the plasma-treated 1-PFC-treated glass slide with PFC oil (**FIG. 21B(ii)**).

This process was again repeated with plasma-treated 4-PFC-treated soda lime glass slides. As shown in **FIG. 22**, two slides of soda lime glass were modified with an oxygen plasma treatment for 40 seconds and silanized (∼2 hours) (nonafluorohexyltrichlorosilane, Gelest, SIN6597.6) to achieve 4-fluoridated carbon-treated glass. One glass slide was coated with 250 µL PFC oil (Sigma Fluorinert® FC-70, Product Number F9880), and 75 microliters of anticoagulant-free blood was pipetted onto both slides. The surfaces were imaged, then tilted by hand and immediately imaged again as shown in **FIG. 22B****.** The blood adhered to the plasma-treated 4-PFC-treated glass side without PFC oil (**FIG. 22B(i)**). In contrast, no blood adhered to the plasma-treated 4-PFC-treated glass slide with PFC oil (**FIG. 22B(ii)**).

Similarly, the ability of 6-PFC-treated glass sides with and without PFC oil to repel blood was compared. This process was again repeated with plasma-treated 4-PFC-treated soda lime glass slides. As shown in **FIG. 23**, two slides of soda lime glass were modified with an oxygen plasma treatment for 40 seconds and silanized (∼2 hours) (Tridecafluorotetrahydrooctyltrichlorosilane, Gelest, SIT8174.0) to achieve 6-fluoridated carbon-treated glass. One glass slide was coated with 250 µL PFC oil (Sigma Fluorinert® FC-70, Product Number F9880), and 75 microliters of anticoagulant-free blood was pipetted onto both slides. The surfaces were imaged, then tilted by hand and immediately imaged again as shown in **FIG. 23B**. The blood adhered to the plasma-treated 6-PFC-treated glass side without PFC oil (**FIG. 23B(i)**). In contrast, no blood adhered to the plasma-treated 6-PFC-treated glass slide with PFC oil (**FIG. 23B(ii)**).

Likewise, when this process was repeated with 8-PFC-treated glass slides, which were silanized for 2 hours (Heptadecafluorotetrahydrodecyltrichlorosilane, Gelest, SIH5841.0), blood adhered to the plasma-treated 8-PFC-treated glass side without PFC oil (**FIG. 24B(i)**), but none adhered to the plasma-treated 8-PFC-treated glass slide with PFC oil (**FIG. 24B(ii)**).

### Example 16

Experiments were conducted to determine whether oleophilic surface and hydrocarbon oil can be used to create a slippery surface capable of repelling blood. As a control, two soda lime glass slides were modified with an oxygen plasma treatment for 40 seconds. One glass slide was coated with 250 µL of light mineral oil (Sigma, M8410), and 75 µL of human blood without anticoagulant was applied to both slides. The surfaces were imaged, tilted by hand, and immediately imaged again (**FIG. 25B**). Blood adhered to both glass slides without surface deposition (see, **FIG. 25B(i)** and **25B(ii)**).

Referring to **FIG. 26**, soda lime glass slides were modified with an oxygen plasma treatment for 40 seconds, and then silanized for 2 hours (trichloro(octyl)silane, Sigma, 235725) (**FIG. 26C**). One glass slide was coated with 250 µL of light mineral oil (Sigma, M8410), and 75 µL of human blood without anticoagulant was applied to both slides. The surfaces were imaged, tilted by hand, and immediately imaged again (**FIG. 26B**). Blood adhered to the 8-HC-treated glass slide (**FIG. 26B(i)**, but none adhered to the 8-HC-treated glass slide treated with mineral oil (see, **FIG. 26B(ii)**).

**FIG. 28** shows unmodified, soda lime glass slides without plasma treatment, which compare untreated glass, glass with PFC oil (Fluorinert FC-70 (Sigma, F9880)), and glass with 250 µL of light mineral oil (Sigma, M8410). No slides were silanized. Seventy-five µL of human blood without anticoagulant was applied to each slide. The surfaces were imaged, tilted by hand, and immediately imaged again (**FIG. 28B**). Blood adhered to the unsilanized soda lime glass (**FIG. 28B(i)**), unsilanized glass treated with PFC oil (**FIG. 28B(ii)**), and unsilanized glass treated with light mineral oil (**FIG. 28B(iii)**).

### Example 17

The disclosed methods can be used to prevent blood from coagulating or adhering to glass beads. **FIG. 29** shows three images of silanized 1 mm glass beads. The beads were subjected to 1 hour of sonication in soapy water, and 1 M of sodium hydroxide is added for 1 hour. The beads were silanized in 5 % v/v in ethanol (nonafluorohexyltrichlorosilane, Gelest, SIN6597.6) and PFC oil (Fluorinert FC-70) was added to create an ultra-slippery surface on the beads (i). Twenty mL of human blood without anticoagulant was added to the beads (ii). The beads, rinsed with PBS solution, showed little to no adhesion of blood material (iii).

Similarly, **FIG. 30** shows washed, unmodified glass beads that had been exposed to anticoagulant-free blood, which formed a solid clot around the beads (i). Silanized, PFC oil coated beads on which blood had been pipetted and washed with PBS showed small amounts of adhesion of blood material on the beads (ii). Silanized beads with a PFC oil coating before exposure to anticoagulant-free blood showed no blood on the beads (iii).

### Example 18 (not according to the present disclosure)

**FIG. 32** shows a commercial application of the liquid repellent surfaces described herein. Two commercially available ketchup bottles made of PETE were emptied of ketchup, rinsed with deionized water and ethanol, successively, and baked at 60 C for 18 hours. Nonafluorohexyltrichlorosilane (Gelest, Product number SIN6597.6) was vapor deposited onto the inner surface of one bottle for 5 hours. The inside of one silanized bottle was coated with PFC oil (FC-70, Sigma), and the excess oil was poured out Ketchup was poured back into both bottles. The contents of the treated and untreated bottles were poured into Erlenmeyer flasks (**FIG. 32A**) and were allowed to rest vertically (**FIG. 32B**). After 10 minutes, the untreated, unmodified bottle showed significant adhesion of ketchup on the walls of the bottle (**FIG. 32B(i)**). However, little to no ketchup adhered to the silanized ketchup bottle treated with PFC oil (**FIG. 32B(ii)**).

### Example 19

The following exemplary experiment conditions and procedures can be used to test the robustness and stableness of the surface treatment in some embodiments:
1) 1/4" medical grade PVC tubes (Sorin Group #020463101) were plasma treated under 170 mTorr oxygen for 120 sec at 100 W and liquid silanized using 5% tridecafluoro-1,1,2,2-hydrooctyl trichlorosilane (Gilest #SIT8174.0) in ethanol (%v/v) for 1 hour and dried overnight at 60 degrees C;
2) One half of the PVC tubes above were packaged in Self-Seal Sterilization Pouches (Cardinal Health #92713) and sterilized using ethalene oxide under standard hospital sterilization protocols. The other half of the PVC tubes above were unsterilized and stored covered at room temperature;
3) Seven days later, the sterilized and non-sterilized tubes were coated with either Fluorinert FC-70 (3M) or perfluordecalin (sigma) and allowed to drain briefly;
4) Pig blood that was collected into a CPD bag with 100 unit/kg of Heparin was filtered using a 40um cell strainer (BD) and recalcified with 7.5mM calcium chloride/magnesium chloride (100mM CaC12, 75mM MgC12) and deheparinized with 3.5mg/ml protamine sulphate. 30mL was then flowed through the tubes at 30ml/min driven by a syringe pump;
5) The blood was then gravity drained from the tubing and the tubing was imaged to compare treated vs. untreated tubing and sterilized vs. non sterilized tubing. (Untreated control tubes were not silanized, sterilized or coated with perfluorocarbon oil, but was otherwise handled the same as the treated tubes.)

**FIGS. 33A-E** demonstrate the test results of a series of blood residue experiments (e.g., as described in the above procedures and conditions) using untreated or treated medical grade PVC tubes. **FIG. 33A** demonstrates that significant blood residues remain inside untreated tubes. **FIG. 33B** demonstrates that blood residues are significantly reduced inside FC-70 treated (non-sterilized) tubes. **FIG. 33C** demonstrates that the repellant characteristics are largely unaffected by sterilization. **FIG. 33D** demonstrates that blood residues are significantly reduced inside PFD treated (non-sterilized) tubes. **FIG. 33E** demonstrates that the repellant characteristics are largely unaffected by sterilization.

### Example 20

Silicon wafer from University Wafer, acrylic (PMMA) from McMaster Carr, and polysulfone from McMaster Carr were plasma treated for 1 min using oxygen plasma at 160 mTorr and 100W. Silane treatment was then carried out for 1 hour with 5% (tridecafluoro-1,1,2,2-tetrhydroocyl) trichlorosilane (v/v) with pure ethanol (anhydrous), followed by rinses of ethanol, deionized water, and ethanol and by a 65 °C bake for 2 hours. 5 µL of water was placed on each sample with a pipette and average and standard deviation were measured using 3 images for each sample. As shown in **FIG. 34**, the contact angle decreases after plasma treatment but increases after silanization.

### Example 21

Acrylic (PMMA) from McMaster Carr, ¼ in thick was plasma treated for 1 min using oxygen plasma at 160 mTorr and 100W. Silane treatment was then carried out for 1 hour with 5% (tridecafluoro-1,1,2,2-tetrhydroocyl) trichlorosilane (v/v) with pure ethanol (anhydrous), followed by rinses of ethanol, deionized water, and ethanol, followed by a 65 °C bake for 2 hours. Atomic Force Microscopy (AFM) surface measurements of acrylic surfaces before and after plasma treatment and silanization was carried out acrylic surfaces (Mcmaster Carr), where the mean and standard deviation of root mean squared surface roughness was carried out. Acrylic surface was plasma treated for 60 seconds under 150 mTorr of oxygen gas at 100 W, and silanized for 1 hour with 5% (tridecafluoro-1,1,2,2-tetrhydroocyl) trichlorosilane (v/v) in pure ethanol (anhydrous), followed by rinses of ethanol, deionized water, and ethanol and by a 65 °C bake for 2 hours. The surface roughness decreases from 3.4 nm ± 1.1 nm to about 2.0 ± 0.2 nm after silanization.

The silanized acrylic samples were dipcoated with perfluorodecalin (FluoroMed) and the tilt angle of water, hexadecane, and human blood with citrate was measured by placing 5 µL of the liquid on the prepared sample with pipette. Then, the samples were tilted manually with a goniometer (Edmund Scientific). Samples which did not move the liquid after tilt angles reached 10 degrees were not tilted any further and recorded with a tilt angle of 10 degrees..

**FIG. 35** shows that while the liquids did not move on untreated samples even at 10 degree tilt angle, surfaces treated with the perfluorodecalin shows tilt angles that were below 2 degrees.

### Example 22

Biological characterization before and after treatment with the lubricating liquid was carried out using *in vitro* studies. Polysulfone surface was plasma treated for 60 seconds under 150 mTorr of oxygen gas at 100 W, and silanized for 1 hour with 5% (tridecafluoro-1,1,2,2-tetrhydroocyl) trichlorosilane (v/v) in pure ethanol (anhydrous), followed by rinses of ethanol, deionized water, and ethanol and by a 65 °C bake for 2 hours. "No PFC Oil" samples were plasma/silanized but did not have any lubricating liquid added thereafter. "Perflubron," "Perfluorodecalin," and "Fluorinert FC-70" samples were dipcoated in the respective lubricant and the excess removed by gravity immediately prior to exposure to blood. Samples were photographed after saline rinse. Blood was obtained with informed consent from healthy, male volunteers who had not taken aspirin within 2 weeks of donation and who did not smoke. Blood was drawn in accordance with the Declaration of Helsinki with approval from the Harvard Committee on Human Studies (Protocol Number M20403-101). Blood tilt experiments were conducted at an angle of 90 degrees..

The rate of thrombosis from whole human blood on slippery PMMA and polysulfone was investigated and quantified the adhesion by spiking the blood with fluorescent fibrinogen. Reduced surface adhesion and fibrin formation was observed on all slippery surfaces investigated over untreated surfaces. For blood adhesion experiments, polysulfone or poly(methyl methacrylate) (PMMA) pieces (11mm x 8mm) were incubated for 30, 60 or 90 minutes with heparinized blood (0.25U/ml) containing 15 ug/mL of fluorescent fibrinogen in wells blocked with BSA (1% (w/v)). Time course of thrombus accumulation from slightly heparinized human blood on polysulfone and PMMA in BSA-blocked polystyrene well plates over 30 min, 60 min and 90 min is shown in **FIG. 36****.** As shown, samples treated with perfluordecalin and FC70 showed the most effective reduction in thrombus accumulation.

**FIG. 37** shows the scanning electron microscope images for untreated polysulfone and that which was plasma treated, silanized, and coated with FC-70 after 30 minutes. Similarly to **FIG. 36**, sample treated with FC-70 showed significant reduction in thrombus accumulation.

The time course of thrombus accumulation from slightly heparinized human blood was further observed with fluorescent fibrinogen on acrylic in BSA-blocked polystyrene well plates over 90 minutes. The samples were measured by fluorescence microscopy after saline rinse and images were analyzed with ImageJ softwared. As shown in **FIG. 38**, samples treated with FC-70 showed the least amount of fibrinogen-coated areas. Chemical surface modification significantly decreased fibrin adhesion from untreated PMMA (P<0.001). Fibrin formation was reduced by 97% on slippery PMMA with FC-70 after 90 min, which was statistically significant (P<0.001).

### Example 23

Slippery surface modification and coating with medical grade perfluorinated liquids reduced the thrombogenicity of surfaces when in contact with non-anticoagulated blood in vitro studies.

PVC tubing (Tygon 3603) was plasma treated under 170 mTorr oxygen for 120 sec and liquid silanized (5% tridecafluoro-1,1,2,2-hydrooctyl trichlorosilane in Ethanol) for 1 hour, rinsed with ethanol, deionized water, and ethanol 3X, and dried overnight at 60 degrees C. Half of the PVC tubes were packaged and sterilized by ethylene oxide sterilization. The rest were stored covered at room temperature.

Seven days later the sterilized and non-sterilized tubing was lubricated with perfluordecalin (FluoroMed). Untreated and Pig blood was filtered through 40um cell strainer before 46.9ul/ml 100mM CaCl2/75mM MgCl2 (3/4) and 3.5ug protamine sulphate (1/2) was added to pig blood.

30ml pig blood was flowed through the tubing at 30mL/min using a syringe pump. The blood was re-activated with 100mM CaCl2 and 75mM MgCl2 and protamine sulphate 10ug/U. Arteriovenous shunts were established in the femoral artery and vein of Yorkshire swine using 8F catheters (Medtronic) and ¼" tubing (Sorin Group). The tubing was emptied of blood by gravity and imaged immediately horizontally. As shown in **FIG. 38**, sterilized and unsterilized tubing with perfluorodecalin did not retain blood, while the untreated tubing did. Accordingly, sterilization does not appear to affect the silanization and surfaces remain slippery.

### Example 24

Slippery surface modification and coating with medical grade perfluorinated liquids reduced the thrombogenicity of surfaces when in contact with non-anticoagulated blood in vivo studies.

Biological characterization before and after treatment with the lubricating liquid was carried out using *in vivo* studies. Medical grade plastics with and without the slippery surface treatment were carried out by forming an arteriovenous (AV) bridge between the femoral artery and vein of 40 kg pigs. 8Fr Cannjulae was joined by 24 x ¼ inch PVC tubing. Blood flow at approximately 1L/min (60L/hr) was tested for 8 hours *without use of added anticoagulants.* The slippery shunt remained unobstructed (patent) over 8 hours of ∼1 L/min of blood flow, while the untreated shunt occluded completely within 90 minutes.

Medical grade PVC tubing (Sorin group) was plasma treated under 170 mTorr oxygen for 180 sec and liquid silanized (5% tridecafluoro-1,1,2,2-hydrooctyl trichlorosilane in Ethanol) for 1 hour, rinsed with ethanol, deionized water, and ethanol 3X, and dried overnight at 60 degrees C.

Similarly, medical grade polyurethane catheters were plasma treated under 170 mTorr oxygen for 120 sec and liquid silanized (5% tridecafluoro-1,1,2,2-hydrooctyl trichlorosilane in Ethanol) for 1 hour, rinsed with ethanol, deionized water, and ethanol 3X, and dried overnight at 60 degrees C.

Both samples were sterilized by ethylene oxide before lubrication with perfluorodecalin.

As shown in **FIG. 39A**, the medical grade cannulae and PVC bridge without the lubricating liquid applied thereon shows significant clotting/obstruction, particularly in the cannula:bridge connectors. Occlusion occurred within 1 to 1.5 hours. In contrast, as shown in **FIG. 39B**, when a slippery surface formed on the medical grade cannulae and PVC bridge, minimal clotting and minimal platelet activation is observed even after 8 hours.

As shown in **FIG. 40**, the catheter and tubing were sectioned and imaged. As shown, the samples with the slippery surface showed reduced clotting compared to the untreated control samples.

Moreover, as shown in **FIG. 41**, the samples were weighed while filled with saline and after the saline was emptied to determine the weight of thrombus in the circuit. Less thrombus accumulation was observed in the treated samples than in the control samples

### Example 25

Metal Surface Modification with carboxyl-terminated perfluoropolyethers as an anchoring layer is demonstrated. Krytox 157 FSH (carboxyl terminated poly(hexafluoropropylene oxide), MW 7000-7500, Miller Stephenson) was used as an anchoring layer. FC-70 (Aldrich, lot #MKBF9431V) or Krytox 10x were used as lubricants. Al alloy 6061-T6 was used as a substrate. 30% hydrogen peroxide (Aqua Solutions), absolute ethanol (Pharmco), HFE-7100 (mixture of methyl nonafluorobutyl ether, 30-50%, and methyl nonafluoroisobutyl ether, 70-50%, Miller Stephenson), were used as received. Water used for washes was of Millipore grade.

The representative roughness and waviness data of the flat Al sample are presented in the Table 2.

**Table 2. Roughness and waviness data measured for a flat Al sample**

| **Sample** | **Average Roughness Ra µm** | **RMS Roughness Rq µm** | **Average Waviness Wa µm** | **RMA Waviness Wq µm** |
|---|---|---|---|---|
| Al 6061-T6 flat | 0.3016 | 0.4100 | 0.2848 | 0.3595 |
| Calibration Si Mech. Grade | 0.001975 | 0.000247 | 0.00328 | 0.003975 |

Aluminum plates were sonicated for 30 min sequentially in 30% H₂O₂, water, and absolute ethanol, and then dried in an oven in the air at 100°C for 30 min.

The pre-cleaned samples were put vertically in a Teflon holder and then placed into a 500-mL three-neck flask, equipped with a reflux condenser, thermocouple, heating mantle and nitrogen blanket (bubbler). The flask was charged with a 3 mM solution of Krytox-157FSH in HFE-7100 (8.46 g in 370.5 mL). The solution fully covered the plates as seen in Fig. 1b. The mixture was refluxed under nitrogen at 60°C for 3 h, following which the mixture was let to cool down to room temperature, samples were removed, rinsed sequentially in 40 mL of HFE-7100 and 40 mL of absolute ethanol, and dried in an oven in the air at 80°C for 55 min. Two samples at a time were treated and the solution and the rinses were reused in the treatment of the next sets of samples.

The contact angle measurements were performed at room temperature using a CAM101 (KSV Instruments LTD) instrument and Millipore grade water. The presented values are left, right and average angles for each location. For each sample one to three locations were tested. The samples were held horizontally during the measurements. The representative contact angle data are presented in the Table 3.

**Table 3. Contact angle data measured for the surface-functionalized Al 6061-T6 sample**

| **Sample** | **CA (L), deg** | **CA(R)**, **deg** | **CA(M), deg** | **Comment** |
|---|---|---|---|---|
| Center | 120.217 | 119.267 | 119.742 | a) |
| Edge | 109.751 | 110.147 | 109.949 | a) |

| | | | | |
|---|---|---|---|---|
| a) The sample after the reflux was left overnight at room temperature in the reaction mixture | | | | |

Surface-pretreated aluminum coupon was infused with FC-70 (Aldrich, lot #MKBF9431V) by placing a total of 60 µL (∼130 mg) of FC-70 on the surface of the sample. The FC-70 was allowed to spread for several minutes. The sample was wetted with the lubricant quite readily, resulting in a smooth shiny surface.

To test the surface of the treated sample for liquid repellency, a single drop of water (30 µL, Millipore) was placed on the aluminum surface, and the behavior of the water droplet was observed and videotaped while the surface was tilted in various directions. It was clearly evident that the water droplet slides easily on the functionalized and lubricated surface, with very little resistance, at low tilt angles, and without pinning.

The functionalization chemical reaction is shown below. Post functionalization, the flat chemically functionalized sample exhibited contact angle 110-120 deg, close to the maximum reported water contact angle on flat PTFE surface - -120 deg. The observed value indicates that the functionalization did occur.

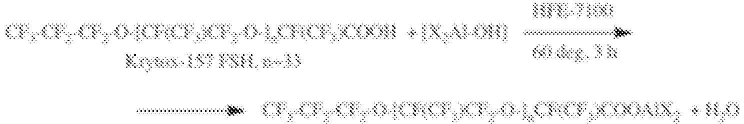

As expected, the fluorinated lubricant FC-70 spread easily on the functionalized substrate, creating a smooth slippery surface that exhibited fluid-repelling behavior, as evidenced by the free movement of the droplet on the surface at low tilt angles, without resistance, and with no pinning.

### Example 26

Chemical functionalization of a solid substrate with a phosphonic acid ligand to match the affinity with a liquid lubricant to form slippery surfaces is demonstrated.

All chemicals were purchased from Sigma-Aldrich and used without further purification unless specified otherwise.

Solution-phase chemical functionalization of metal oxide substrates using perfluorinated phosphonic acid ligand for matching the affinity of substrate with lubricant

In order to create a monolayer of fluoroalkyl chains on aluminum oxy hydroxide substrate surface, we submerged samples in a 1 wt. % solution of *1H,1H,2H,2H*-perfluorooctyl phosphonic acid (F13PA) or FS100 (fluoroaliphatic phosphate ester fluorosurfactant, Mason Chemical Company) in 95:5 ethanol:water for 1 h at 70°C. In another aspect, substrates having a portion that can be damaged at elevated temperature were fluorinated in the same bath at lower temperature for a longer period of time (e.g. 3-4 h at 40°C or overnight at room temperature). In yet another aspect, substrates having a portion that can be damaged by alcohols (e.g. PMMA, medical grade PVC) were fluorinated in an aqueous solution of FS100 prepared in the presence of 1 wt. % Pluronic F-68 (EO₇₈PO₃₀EO₇₈, FW=8400, Affymetrix) to dissolve FS100, then following the same procedure as for other substrates.

Substrates that are not compatible with solution-phase chemical functionalization methods (e.g. PDMS, PHEMA hydrogel) were fluorinated using C₄F₈ plasma using an inductively coupled plasma reactive ion etching system (STS MPX/LPX RIE) with C₄F₈ flow rate of 120 seem (standard cubic centimeter per minute) for 8 sec under 1 mTorr pressure and 600 W/0 W coil/platen power.

All the fluorinated surfaces were lubricated by application of a perfluoropolyether (PFPE) lubricant (DuPont Krytox GPL 100, "K100"). To spread out the lubricant, the substrates were either tilted or spun on a spin coater. Excess lubricants were typically removed by spinning the substrates at higher spin rate (>3,000 rpm, 1 min) or by pressure washing the substrate in a stream of high-pressure water.

**FIG. 42** shows through FTIR spectra that slippery surfaces were successfully formed as evidenced by the characteristic peaks that arise through the four different stages of functionalization from bare aluminum (Al), aluminum oxy hydroxide (Al-B), fluoro-functionalized aluminum oxy hydroxide (Al-BF), and pure fluoroaliphatic phosphate ester fluorosurfactant (FS100).

### Example 27

Glass bottom 24 well plates (Matek Corporation, P24G-0-13-F) were plasma and silane treated with or without PFD (which was added and then pipetted off). Then, 1ml of a 0.5ug/ml solution of fluorescent fibrinogen Alexa Fluor 647 (Invitrogen, F35200) in phosphate buffered saline was added to each well. Images were taken on a Leica TIRF on DM16000B using a 63x oil objective with multipoint positioning.

As shown in **FIG. 43** (scale bar = 20um), fibrinogen molecules in saline are repelled from slippery glass. Fluorescent fibrinogen particles (see arrows) stick to glass without perfluorocarbon (top) but do not stick to surface and continue to move over glass treated with the perfluorocarbon material (bottom).

### Example 28

Glass bottom 24 well plates (Matek Corporation, P24G-0-13-F) were plasma and silane treated with or without Vitreon (which was added and then pipetted off). Then, 1ml heparinized whole human blood, spiked with 1.5ug/ml solution of fluorescent fibrinogen Alexa Fluor 647 (Invitrogen, F35200), was added to each well. Images were taken with a Leica SP5 X MP Inverted Confocal Microscope using a 20X objective with multipoint positioning.

As shown in **FIG. 44** (scale bar = 100um), whole blood repelled from slippery glass. Fluorescent thrombi (arrows) stick to glass without perfluorocarbon (top) but do not stick to surface and continue to move over glass treated with the perfluorocarbon material (bottom).

### Example 29

The ability of functionalized filter paper treated with PFC oil to repel liquid was investigated and compared to that of untreated and unmodified filter paper, filter paper treated with PFC oil, and silanized filter paper. Two pieces of filter paper (Whatman, #B-2, 10347673) were further modified with an oxygen plasma treatment at 170 mTorr for 60 seconds at 200 W. Those two filters were silanized overnight with trichloro(1H,1H,2H,2H-perfluorooctyl)silane under vacuum for approximately 13 hours.

Seventy-five µL of perfluorodecalin were applied to one silanized and one unsilanized pieces of filter paper to create "PFC-oiled" surfaces. Twenty-five µL of human blood were applied to all four surfaces.

The surfaces were imaged, then tilted by hand and immediately imaged again. The blood adhered to untreated and unmodified filter paper, the filter paper treated with PFC oil, and the silanized filter paper, but was completely repelled by silanized filter paper with PFC oil.

### Example 30

The ability of a functionalized glass fiber filter treated with PFC oil to repel liquid was investigated and compared to that of a untreated and unmodified glass fiber filter, a glass fiber filter treated with PFC oil, and a silanized glass fiber filter. Two glass fiber filters (Millipore, AP2007500) were further modified with an oxygen plasma treatment at 170 mTorr for 60 seconds at 200 W. Those two filters were silanized overnight with trichloro(1H,1H,2H,2H-perfluorooctyl)silane under vacuum for approximately 13 hours.

Seventy-five µL of perfluorodecalin were applied to one silanized and one unsilanized glass fiber filter to create "PFC-oiled" surfaces. Twenty-five µL of human blood were applied to all four surfaces.

The surfaces were imaged, then tilted by hand and immediately imaged again. The blood adhered to untreated and unmodified glass fiber filter, the glass fiber filter treated with PFC oil, and the silanized glass fiber filter, but was completely repelled by silanized glass fiber filter with PFC oil.

### Example 31

The composition of the leaving group on the silane and the composition of the solvent during the silanization was investigated. Perfluorodecalin was used as the solvent for deposition of the silane. The ability of acrylic functionalized with either triethoxysilane or trichlorosilane and further treated with PFC oil to repel liquid was investigated and compared to that of untreated and unmodified acrylic, acrylic treated with PFC oil, and acrylic functionalized with either triethoxysilane or trichlorosilane. Six sheets of Clear Cast Acrylic Sheet, 0.060" Thick, (McMaster Carr, Product Number 8560K 171) were obtained. Two acyrylic sheets were silanized in perfluorodecalin with five volume percent trichloro(1H,1H,2H,2H-perfluorooctyl)silane (Gelest, SIT8174.0) for 1 hour. Two separate filters were silanized in perfluorodecalin (FluoroMed, AP140-HP) with five volume percent triethoxy (1H,1H,2H,2H-perfluorooctyl)silane (Gelest, SIT8175.0) for 1 hour. The four silanized sheets were rinsed with 1 milliliter of perfluorodecalin, dried with compressed air, and baked at 60 degrees Celsius for 2 hours.

Seventy-five µL of perfluorodecalin were applied to one silanized with trichlorosilane, on silanized with triethoxysilane, and one unsilanized acrylic sheet to create "PFC-oiled" surfaces. Twenty-five µL of human blood were applied to all six surfaces.

The surfaces were imaged, then tilted by hand and immediately imaged again. The blood adhered to untreated and unmodified acrylic, acrylic treated with PFC oil, and acrylic functionalized with either triethoxysilane or trichlorosilane., but was completely repelled by acrylic functionalized with either triethoxysilane or trichlorosilane and further treated with PFC oil.

A desired functionality, intended to achieve certain medically relevant response (such as anti-clotting, blood or other biological fluid repelling, drug releasing, infection-suppressing, tissue growth promoting, etc.), can be engineered into the composition of the anchoring and lubricating layers. The particular embodiments described above are, therefore, to be considered as illustrative and not restrictive. Those skilled in the art will recognize, or be able to ascertain, using no more than routine experimentation, numerous equivalents to the specific embodiments described herein. The scope of the invention is as set forth in the appended claims and equivalents thereof, rather than being limited to the examples contained in the foregoing description.

## Claims

1. A medical device having a slippery surface, comprising:
a smooth substrate comprising a monomolecular fluorous anchoring layer; the anchoring layer comprising;
a head group covalently attached to the substrate and a functional group directly or indirectly attached to the head group; and
a lubricating layer comprising a perfluorocarbon oil having an affinity for the functional group and disposed over the anchoring layer, wherein the anchoring layer and the lubricating layer are held together by non-covalent attractive forces,
wherein the anchoring layer and the lubricating layer form a surface configured and arranged for contact with a material that is substantially immiscible with the perfluorocarbon oil, wherein the surface repels the material that is substantially immiscible with the perfluorocarbon oil;
wherein the medical device is selected from the group consisting of catheters, shunts, blood dialysis instruments, extracorporeal membrane oxygenation machines, heart lung machines, ventilator associated ventilator tubing, artificial organs, organ implants, knee and hip replacement implants, cochlear implants, dental implants, endoscopes, and combinations thereof.

2. The medical device of claim 1, wherein said head group of the anchoring layer includes ethers, silyl ethers, siloxanes, esters of carboxylic acids, esters of sulfonic acids, esters of sulfinic acids, esters of sulfuric acids, esters of phosphonic acids, esters of phosphinic acids, esters of phosphoric acids, silyl esters of carboxylic acids, silyl esters of sulfonic acids, silyl esters of sulfinic acids, silyl esters of sulfuric acids, silyl esters of phosphonic acids, silyl esters of phosphinic acids, silyl esters of phosphoric acids, oxides, sulfides, carbocycles, heterocycles with at least one oxygen atom, heterocycles with at least one nitrogen atom, heterocycles with at least one sulfur atom, heterocycles with at least one silicon atom, 'click' reactions-derived heterocycles, Diels-Alder reactions-derived carbocycles, Diels-Alder reactions derived heterocycles, amides, imides, sulfides, thiolates, metal thiolates, urethanes, oximes, hydrazides, hydrazones, or combinations thereof.

3. The medical device of any of claims 1-2, wherein the functional group of the anchoring layer comprises hydrocarbons, partially fluorinated hydrocarbons, perfluorocarbons, perfluorooligoethers, or perfluoropolyethers.

4. The medical device of any preceding claim, wherein the slippery surface is hydrophobic or omniphobic.

5. The medical device of any of claims 1-4, wherein the anchoring layer comprises a silyl ester or silyl ether group covalently attached to a hydrocarbon, partially fluorinated hydrocarbon, and/or perfluorocarbon tail, and wherein the anchoring layer and the lubricating layer form a hydrophobic or an omniphobic surface.

6. The article of claim 5, wherein, the anchoring layer comprises a silyl ester or silyl ether group covalently attached to a partially fluorinated hydrocarbon, and/or perfluorocarbon tail.

7. The medical device of any of claims 1-4, wherein the anchoring layer comprises a phosphonate or carboxylate group covalently attached to a hydrocarbon, partially fluorinated hydrocarbon and/or perfluorocarbon tail, and wherein the anchoring layer and the lubricating layer form a hydrophobic or an omniphobic surface.

8. The medical device of any preceding claim, wherein the article resists colonization by bacteria selected from the group consisting of: *Staphylococcus, Streptococcus, Enterobacter,* or *Pseudomonas.*

9. The medical device of any preceding claim, wherein the substantially immiscible material is selected from whole blood, plasma, serum, cerebrospinal fluid, peritoneal fluid, nasopharyngeal fluid, wound exudate, or combinations thereof.

10. The medical device of any preceding claim, wherein the medical device is a catheter or a shunt.

11. The medical device of any preceding claim, wherein catheter selected from the group consisting of: central lines, peripherally inserted central catheter (PICC) lines, urinary catheters, vascular catheters, peritoneal dialysis catheters, and central venous catheters.

12. The medical device of any preceding claim, wherein the medical device is an endoscope.

13. The medical device of any preceding claim, wherein the endoscope is selected from the group consisting of: hysteroscopes, cystoscopes, amnioscopes, laparoscopes, gastroscopes, mediastinoscopes, bronchoscopes, esophagoscopes, rhinoscopes, arthroscopes, procloscopes, colonoscopes, nephroscopes, angioscopes, thoracoscopes, esophagoscopes, laryngoscopes, and encephaloscope.

14. A method of preventing adhesion, adsorption, surface-mediated clot formation, or coagulation of a material on a medical device comprising:
providing a medical device of any preceding claim comprising a slippery surface; and
contacting a material selected from whole blood, plasma, serum, cerebrospinal fluid, peritoneal fluid, nasopharyngeal fluid, wound exudate, or combinations thereof, with the slippery surface;
thereby prevent adhesion, adsorption, surface-mediated clot formulation, or coagulation of the material on the slippery surface of the medical device.

## Patentansprüche

1. Medizinisches Vorrichtung, die eine glatte Oberfläche aufweist, umfassend:
ein glattes Substrat, das eine monomolekulare fluorhaltige Verankerungsschicht umfasst; wobei die Verankerungsschicht umfasst;
eine kovalent an das Substrat gebundene Kopfgruppe und eine direkt oder indirekt an die Kopfgruppe gebundene funktionelle Gruppe; und
eine Gleitschicht, die ein Perfluorkohlenstofföl umfasst, das eine Affinität zur funktionellen Gruppe aufweist, und über der Verankerungsschicht angeordnet ist, wobei die Verankerungsschicht und die Gleitschicht durch nichtkovalente Anziehungskräfte zusammengehalten werden;
wobei die Verankerungsschicht und die Gleitschicht eine Oberfläche bilden, die für den Kontakt mit einem Material konfiguriert und angeordnet ist, das mit dem Perfluorkohlenstofföl im Wesentlichen unvermischbar ist, wobei die Oberfläche das Material abstößt, das mit dem Perfluorkohlenstofföl im Wesentlichen unvermischbar ist;
wobei die medizinische Vorrichtung aus der Gruppe bestehend aus Kathetern, Shunts, Blutdialyseinstrumenten, extrakorporalen Membranoxygenierungsmaschinen, Herzlungenmaschinen, Beatmungsgerät, zugehörigem Beatmungsschlauch, künstlichen Organen, Organimplantaten, Knie- und Hüftersatzimplantaten, Cochleaimplantaten, Zahnimplantaten, Endoskopen und Kombinationen davon ausgewählt ist.

2. Medizinische Vorrichtung nach Anspruch 1, wobei die Kopfgruppe der Verankerungsschicht Ether, Silylether, Siloxane, Carbonsäureester, Sulfonsäureester, Sulfinsäureester, Schwefelsäureester, Phosphonsäureester, Phosphinsäureester, Phosphorsäureester, Carbonsäure-Silylester, Sulfonsäure-Silylester, Sulfinsäure-Silylester, Schwefelsäure-Silylester, Phosphonsäure-Silylester, Phosphinsäure-Silylester, Phosphorsäure-Silylester, Oxide, Sulfide, Carbocyclen, Heterocyclen mit mindestens einem Sauerstoffatom, Heterocyclen mit mindestens einem Stickstoffatom, Heterocyclen mit mindestens einem Schwefelatom, Heterocyclen mit mindestens einem Siliciumatom, von Klickreaktionen abgeleitete Heterocyclen, von Diels-Alder-Reaktionen abgeleitete Carbocyclen, von Diels-Alder-Reaktionen abgeleitete Heterocyclen, Amide, Imide, Sulfide, Thiolate, Metallthiolate, Urethane, Oxime, Hydrazide, Hydrazone oder Kombinationen davon umfasst.

3. Medizinische Vorrichtung nach einem der Ansprüche 1 bis 2, wobei die funktionelle Gruppe der Verankerungsschicht Kohlenwasserstoffe, teilweise fluorierte Kohlenwasserstoffe, Perfluorkohlenwasserstoffe, Perfluoroligoether oder Perfluorpolyether umfasst.

4. Medizinische Vorrichtung nach einem der vorhergehenden Ansprüche, wobei die glatte Oberfläche hydrophob oder omniphob ist.

5. Medizinische Vorrichtung nach einem der Ansprüche 1 bis 4, wobei die Verankerungsschicht eine Silylester- oder Silylethergruppe umfasst, die kovalent an einen Kohlenwasserstoff, einen teilweise fluorierten Kohlenwasserstoff und/oder einen Perfluorkohlenwasserstoffschwanz gebunden ist, und wobei die Verankerungsschicht und die Gleitschicht eine hydrophobe oder eine omniphobe Oberfläche bilden.

6. Gegenstand nach Anspruch 5, wobei die Verankerungsschicht eine Silylester- oder Silylethergruppe umfasst, die kovalent an einen teilweise fluorierten Kohlenwasserstoff und/oder einen Perfluorkohlenstoffschwanz gebunden ist.

7. Medizinische Vorrichtung nach einem der Ansprüche 1 bis 4, wobei die Verankerungsschicht eine Phosphonat- oder Carboxylatgruppe umfasst, die kovalent an einen Kohlenwasserstoff-, teilweise fluorierten Kohlenwasserstoff- und/oder Perfluorkohlenstoffschwanz gebunden ist, und wobei die Verankerungsschicht und die Gleitschicht eine hydrophobe oder eine omniphobe Oberfläche bilden.

8. Medizinische Vorrichtung nach einem der vorhergehenden Ansprüche, wobei der Gegenstand der Besiedlung durch Bakterien widersteht, die ausgewählt sind aus der Gruppe bestehend aus: Staphylococcus, Streptococcus, Enterobacter oder Pseudomonas.

9. Medizinische Vorrichtung nach einem der vorhergehenden Ansprüche, wobei das im Wesentlichen unvermischbare Material aus Vollblut, Plasma, Serum, Cerebrospinalflüssigkeit, Peritonealflüssigkeit, Nasopharyngealflüssigkeit, Wundexsudat oder Kombinationen davon ausgewählt ist.

10. Medizinische Vorrichtung nach einem der vorhergehenden Ansprüche, wobei die medizinische Vorrichtung ein Katheter oder ein Shunt ist.

11. Medizinische Vorrichtung nach einem der vorhergehenden Ansprüche, wobei der Katheter ausgewählt ist aus der Gruppe bestehend aus: Zentralkathetern, peripher eingeführten Zentralkatheterleitungen (PICC), Blasenkathetern, Gefäßkathetern, Peritonealdialysekathetern und zentralvenösen Kathetern.

12. Medizinische Vorrichtung nach einem der vorhergehenden Ansprüche, wobei die medizinische Vorrichtung ein Endoskop ist.

13. Medizinische Vorrichtung nach einem der vorhergehenden Ansprüche, wobei das Endoskop ausgewählt ist aus der Gruppe bestehend aus: Hysteroskopen, Zystoskopen, Amnioskopen, Laparoskopen, Gastroskopen, Mediastinoskopen, Bronchoskopen, Ösophagoskopen, Rhinoskopen, Arthroskopen, Proktoskopen, Koloskopen, Nephroskopen, Angioskopen, Thorakoskopen, Ösophagoskopen, Laryngoskopen und Enzephaloskop.

14. Verfahren zur Verhinderung von Adhäsion, Adsorption, oberflächenvermittelter Gerinnselbildung oder Koagulation eines Materials auf einer medizinischen Vorrichtung, umfassend:
Bereitstellen einer medizinischen Vorrichtung nach einem der vorhergehenden Ansprüche, die eine glatte Oberfläche umfasst; und
Inkontaktbringen eines Materials, das aus Vollblut, Plasma, Serum, Cerebrospinalflüssigkeit, Peritonealflüssigkeit, Nasopharyngealflüssigkeit, Wundexsudat oder Kombinationen davon ausgewählt ist, mit der glatten Oberfläche;
dadurch Verhindern von Adhäsion, Adsorption, oberflächenvermittelte Gerinnselformulierung oder Koagulation des Materials auf der glatten Oberfläche der medizinischen Vorrichtung.

## Revendications

1. Dispositif médical possédant une surface glissante, comprenant :
un substrat lisse comprenant une couche d'ancrage fluorée monomoléculaire ; la couche d'ancrage comprenant ;
un groupe de tête lié de manière covalente au substrat et un groupe fonctionnel directement ou indirectement lié au groupe de tête ; et
une couche lubrifiante comprenant une huile perfluorocarbonée présentant une affinité pour le groupe fonctionnel et disposée sur la couche d'ancrage, ladite couche d'ancrage et ladite couche lubrifiante étant maintenues ensemble par des forces d'attraction non covalentes,
ladite couche d'ancrage et ladite couche lubrifiante formant une surface conçue et agencée pour être en contact avec un matériau qui est sensiblement non miscible avec l'huile perfluorocarbonée, ladite surface repoussant le matériau qui est sensiblement non miscible avec l'huile perfluorocarbonée ;
ledit dispositif médical étant choisi dans le groupe constitué par les cathéters, les shunts, les instruments de dialyse sanguine, les machines d'oxygénation à membrane extracorporelle, les cœurs-poumons artificiels, un respirateur, les tubes de respirateur associés, les organes artificiels, les implants d'organes, les implants de remplacement du genou et de la hanche, les implants cochléaires, les implants dentaires, les endoscopes et leurs combinaisons.

2. Dispositif médical selon la revendication 1, ledit groupe de tête de la couche d'ancrage comprenant des éthers, des éthers silyliques, des siloxanes, des esters des acides carboxyliques, des esters des acides sulfoniques, des esters des acides sulfiniques, des esters des acides sulfuriques, des esters des acides phosphoniques, des esters des acides phosphiniques, des esters des acides phosphoriques, des esters de silyle des acides carboxyliques, des esters de silyle des acides sulfoniques, des esters de silyle des acides sulfiniques, des esters de silyle des acides sulfuriques, des esters de silyle des acides phosphoniques, des esters de silyle des acides phosphiniques, des esters de silyle des acides phosphoriques, des oxydes, des sulfures, des carbocycles, des hétérocycles avec au moins un atome d'oxygène, des hétérocycles avec au moins un atome d'azote, des hétérocycles avec au moins un atome de soufre, des hétérocycles avec au moins un atome de silicium, des hétérocycles dérivés de réactions « click », des carbocycles dérivés de réactions de Diels-Alder, des hétérocycles dérivés de réactions de Diels-Alder, des amides, des imides, des sulfures, des thiolates, des thiolates métalliques, des uréthanes, des oximes, des hydrazides, des hydrazones ou leurs combinaisons.

3. Dispositif médical selon l'une quelconque des revendications 1 à 2, ledit groupe fonctionnel de la couche d'ancrage comprenant des hydrocarbures, des hydrocarbures partiellement fluorés, des perfluorocarbures, des perfluorooligoéthers ou des perfluoropolyéthers.

4. Dispositif médical selon l'une quelconque des revendications précédentes, ladite surface glissante étant hydrophobe ou omniphobe.

5. Dispositif médical selon l'une quelconque des revendications 1 à 4, ladite couche d'ancrage comprenant un groupe ester de silyle ou éther silylique lié de manière covalente à un hydrocarbure, un hydrocarbure partiellement fluoré et / ou une queue de perfluorocarbure, et ladite couche d'ancrage et ladite couche lubrifiante formant une surface hydrophobe ou omniphobe.

6. Article selon la revendication 5, ladite couche d'ancrage comprenant un groupe ester de silyle ou éther silylique lié de manière covalente à un hydrocarbure partiellement fluoré et/ou une queue de perfluorocarbure.

7. Dispositif médical selon l'une quelconque des revendications 1 à 4, ladite couche d'ancrage comprenant un groupe phosphonate ou carboxylate lié de manière covalente à une queue d'hydrocarbure, d'hydrocarbure partiellement fluoré et/ou de perfluorocarbure, et ladite couche d'ancrage et ladite couche lubrifiante formant une surface hydrophobe ou omniphobe.

8. Dispositif médical selon l'une quelconque des revendications précédentes, ledit article résistant à la colonisation par des bactéries choisies dans le groupe constitué par : *Staphylococcus, Streptococcus, Enterobacter,* ou *Pseudomonas.*

9. Dispositif médical selon l'une quelconque des revendications précédentes, ledit matériau sensiblement non miscible étant choisi parmi le sang total, le plasma, le sérum, le liquide céphalorachidien, le liquide péritonéal, le liquide nasopharyngé, un exsudat de plaie ou leurs combinaisons.

10. Dispositif médical selon l'une quelconque des revendications précédentes, ledit dispositif médical étant un cathéter ou un shunt.

11. Dispositif médical selon l'une quelconque des revendications précédentes, ledit cathéter étant choisi dans le groupe constitué par : les lignes centrales, les lignes de cathéter central à insertion périphérique (PICC), les cathéters urinaires, les cathéters vasculaires, les cathéters de dialyse péritonéale et les cathéters veineux centraux.

12. Dispositif médical selon l'une quelconque des revendications précédentes, ledit dispositif médical étant un endoscope.

13. Dispositif médical selon l'une quelconque des revendications précédentes, ledit endoscope étant choisi dans le groupe constitué par : les hystéroscopes, les cystoscopes, les amnioscopes, les laparoscopes, les gastroscopes, les médiastinoscopes, les bronchoscopes, les œsophagoscopes, les rhinoscopes, les arthroscopes, les procloscopes, les colonoscopes, les néphroscopes, les angioscopes, les thoracoscopes, les œsophagoscopes, les laryngoscopes et encéphaloscope.

14. Procédé permettant d'empêcher l'adhérence, l'adsorption, la formation de caillots induite par des surfaces, ou la coagulation d'un matériau sur un dispositif médical comprenant :
la fourniture d'un dispositif médical selon l'une quelconque des revendications précédentes, comprenant une surface glissante ; et
la mise en contact d'un matériau choisi parmi le sang total, le plasma, le sérum, le liquide céphalorachidien, le liquide péritonéal, le liquide nasopharyngé, un exsudat de plaie ou leurs combinaisons, avec la surface glissante ;
empêchant ainsi l'adhérence, l'adsorption, la formulation de caillots induite par des surfaces, ou la coagulation du matériau sur la surface glissante du dispositif médical.
